(19) Europäisches Patentamt
European Patent Office
Office européen des brevets

(11) **EP 1 448 168 B1**

(12) **EUROPEAN PATENT SPECIFICATION**

(45) Date of publication and mention of the grant of the patent:
**03.01.2007 Bulletin 2007/01**

(21) Application number: **02779684.6**

(22) Date of filing: **14.11.2002**

(51) Int Cl.:
***A61K 9/16*** *(2006.01)* ***A61K 31/56*** *(2006.01)*

(86) International application number:
**PCT/GB2002/005159**

(87) International publication number:
**WO 2003/043606 (30.05.2003 Gazette 2003/22)**

(54) **PHARMACEUTICAL FORMULATION COMPRISING BICALUTAMIDE**

BICALUTAMID ENTHALTENDE PHARMAZEUTISCHE ZUSAMMENSETZUNG

FORMULATION PHARMACEUTIQUE COMPRENANT DU BICALUTAMIDE

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **16.11.2001 SE 0103839**

(43) Date of publication of application:
**25.08.2004 Bulletin 2004/35**

(73) Proprietor: **AstraZeneca UK Limited**
**London,**
**Greater London W1Y 1LN (GB)**

(72) Inventors:
- **BATEMAN, Nicola, Frances**
  **Macclesfield, Cheshire SK10 4TG (GB)**
- **CAHILL, Julie, Kay**
  **Macclesfield, Cheshire SK10 2NA (GB)**
- **CARMAN, Neill, Hugh**
  **Macclesfield, Cheshire SK10 4TG (GB)**
- **COCKSHOTT, Ian, Derek**
  **Macclesfield, Cheshire SK10 4TG (GB)**

(74) Representative: **Gainey, Laurence David Scott et al**
**AstraZeneca AB,**
**Global Intellectual Property**
**151 85 Sodartalje (SE)**

(56) References cited:
- **DENIS L ET AL: "PHARMACODYNAMICS AND PHARMACOKINETICS OF BICALUTAMIDE: DEFINING AN ACTIVE DOSING REGIMEN" UROLOGY, BELLE MEAD, NJ, US, vol. 47, no. SUPPL 1A, 1996, pages 26-28, XP000605159 ISSN: 0090-4295**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a pharmaceutical product for administration to a patient, the product comprising 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in solid dispersion comprising an enteric polymer having a $pK_a$ from 3 to 6, the product further comprising an anti-oestrogen or an aromatase inhibitor. In one particular embodiment >50% of the 4'-cyano-α',α',α'-trifluoro-3-(4-fluorophenylsulphonyl)-2-hydroxy-2-methylpropiono-*m*-toluidide is provided in the form of the R-enantiomer. The invention also relates to a daily pharmaceutical dose of 4'-cyano-α',α',α'-trifluoro-3-(4-fluorophenylsulphonyl)-2-hydroxy-2-methylpropiono-*m*-toluidide provided by such a formulation. In addition, the invention relates to the use of such an enteric polymer in solid dispersion with 4'-cyano-α',α',α'-trifluoro-3-(4-fluorophenylsulphonyl)-2-hydroxy-2-methylpropiono-*m*-toluidide for increasing the bioavailability of the 4'-cyano-α',α',α'-trifluoro-3-(4-fluorophenylsulphonyl)-2-hydroxy-2-methylpropiono-*m*-toluidide; for reducing inter-patient variability in plasma concentrations of 4'-cyano-α',α',α'-trifluoro-3-(4-fluorophenylsulphonyl)-2-hydroxy-2-methylpropiono-*m*-toluidide; or for treating and/or reducing the risk of prostate cancer in a patient.

## BACKGROUND TO THE INVENTION

Bicalutamide, a non-steroidal anti-androgen, is the racemate of 4'-cyano-α',α',α'-trifluoro-3-(4-fluorophenylsulphonyl)-2-hydroxy-2-methylpropiono-*m*-toluidide and is known by the AstraZeneca trade name CASODEX™. EP-100172 discloses 4'-cyano-α',α',α'-trifluoro-3-(4-fluorophenylsulphonyl)-2-hydroxy-2-methylpropiono-m-toluidide (named in EP-100172 as 4-cyano-3-trifluoromethyl-*N*-(3-*p*-fluorophenylsulphonyl-2-hydroxy-2-methylpropionyl)aniline) as the 8th compound listed in the table in Example 6. The corresponding structure is shown in formula I:-

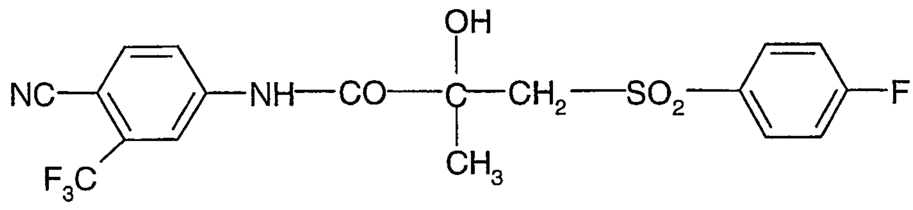

I

Bicalutamide can be used to combat prostate cancer. The properties and usefulness of bicalutamide as an anti-androgen have been reviewed in B J A Furr *et al.,* Urology, 1996, 47 (Suppl. 1A), 13-25, and G J C Kolvenbag *et al.,* Urology, 1996, 47 (Suppl. 1A), 70-79. 4'-cyano-α',α',α'-trifluoro-3-(4-fluorophenylsulphonyl)-2-hydroxy-2-methylpropiono-*m*-toluidide can exist in distinct R- and S- enantiomeric forms. The R-enantiomer is the (-) isomer and is the pharmacologically active compound *in vivo.* For further details of the enantiomers, reference is made to Tucker and Chesterton, *J. Med. Chem.* 31, pp 885-887 (1988).

The chemical synthesis of racemic 4'-cyano-α',α',α'-trifluoro-3-(4-fluorophenylsulphonyl)-2-hydroxy-2-methylpropiono-*m*-toluidide is described in US4636505, and this disclosure is incorporated herein by reference. The R-enantiomer may be obtained by the resolution of enantiomers from the racemate or resolution of precursors of the enantiomers using fractional crystallisation or chromatographic separation of diastereomeric esters of chiral acids. Other methods will, however, be evident to the skilled addressee using routine techniques for the preparation of enantiomers. For example, the R-enantiomer may be prepared by simple crystallisation and chromatographic resolution (see, for example, Wilen and Lochmuller, "Tables of Resolving Agents", *J. Chromatography,* 113, 283-302 (1975) and E L Eliel, *Stereochemistry of Carbon Compounds,* McGraw Hill (1962)). Another method involves resolution of the carboxylic acid precursor, 3-(4-fluorophenyl)-2-hydroxy-2-methylpropanoic acid, by fractional crystallisation of diastereomeric salts with chiral amines. The Tucker and Chesterton reference cited above discloses the chromatographic separation of the R- and S-enantiomers from racemic 4'-cyano-α',α',α'-trifluoro-3-(4-fluorophenylsulphonyl)-2-hydroxy-2-methylpropiono-*m*-toluidide. The method involves the chromatographic separation of R-camphanoyl esters of the racemate and their hydrolysis and oxidation to the R- and S-enantiomers.

This disclosure provides an illustration of a method of obtaining the enantiomers for use in the present invention.

Bicalutamide (4'-cyano-α',α',α'-trifluoro-3-(4-fluorophenylsulphonyl)-2-hydroxy-2-methylpropiono-*m*-toluidide racemate) is used in conventional oral tablet form (eg, at a daily monotherapy dose of 50mg and 150mg) to combat

prostate cancer in men. The bioavailability of the bicalutamide to the patient is determined to a certain extent by the dissolution rate and solubility of the drug in the GI tract, which affects absorption across mucosal membranes in the GI tract. The relative bioavailability of bicalutamide for a series of formulations can be assessed by determining the area under the curve (AUC) of a graph of plasma bicalutamide concentration ν. time elapsed since administration of the bicalutamide. As a consequence of sub-optimal rates of dissolution and degree of solubility of the drug, there is observed a high degree of inter-patient variability in the bioavailability of bicalutamide administered in conventional tablet form. This may result in sub-optimal treatment efficacy in a proportion of patients. In addition, the maximum systemic exposure achievable after dosing the conventional tablet is limited, such that at conventional tablet doses in excess of 150mg, there is a significant reduction in bicalutamide bioavailability.

It would be desirable to extend the therapeutic potential of 4'-cyano-α',α',α'-trifluoro-3-(4-fluorophenylsulphonyl)-2-hydroxy-2-methylpropiono-*m*-toluidide by increasing the bioavailability of the drug and/or reducing inter-patient variability in plasma concentrations of bicalutamide, relative to conventional bicalutamide, as a result of reduced inter-patient variability in the absorption of the drug.

Such increased bioavailability could be useful in enabling a reduction in the daily dose of 4'-cyano-α',α',α'-trifluoro-3-(4-fluorophenylsulphonyl)-2-hydroxy-2-methylpropiono-*m*-toluidide required to achieve the same level of bioavailability seen with a conventional formulation.

A possible benefit of achieving relatively higher bioavailability could also be the ability to extend treatment to more advanced stages of prostate cancer than are currently treated with the conventional formulations. This could be useful, for example, for treating patients with metastatic prostate cancer, using for example 4'-cyano-α',α',α'-trifluoro-3-(4-fluorophenylsulphonyl)-2-hydroxy-2-methylpropiono-*m*-toluidide as a monotherapy (ie, not in combination with LHRH analogue therapy or surgical castration).

As another advantage, it would also be desirable to reduce inter-patient variability in plasma concentrations of 4'-cyano-α',α',α'-trifluoro-3-(4-fluorophenylsulphonyl)-2-hydroxy-2-methylpropiono-*m*-toluidide as a result of reduced inter-patient variability in the absorption of bicalutamide. This would increase predictability of the treatment and increase uniformity of treatment in a patient population.

EP-0988863 deals with the issue of increasing the bioavailability of poorly soluble drugs in general. 4'-cyano-α',α',α'-trifluoro-3-(4-fluorophenylsulphonyl)-2-hydroxy-2-methylpropiono-*m*-toluidide is not specifically addressed. The disclosed solution is to provide a formulation comprising a water-insoluble complex of the drug and a water-insoluble ionic polymer. No specific class of polymer is required, and the polymer can be cationic or anionic, but must have a molecular weight greater than about 80,000 D and a glass transition temperature equal or greater than about 50°C.

EP-1027886 also deals with the issue of increasing the bioavailability of poorly soluble drugs in general. Again, 4'-cyano-α',α',α'-trifluoro-3-(4-fluorophenylsulphonyl)-2-hydroxy-2-methylpropiono-*m*-toluidide is not specifically addressed. The disclosed solution is to provide a solid dispersion formulation comprising a low-solubility drug and a polymer. The latter can be one of many possible polymers, as long as it has a glass transition temperature of at least 100°C measured at 50% relative humidity. Some enteric polymers (eg, HPMCP polymers, including grades HP-50™, HP-55™ and HP-55S™) are explicitly excluded from use, since it is explained that all of these polymers absorb sufficient water upon equilibration at 50% relative humidity that their respective glass transition temperatures drop below 100°C. Hydroxypropyl methylcellulose acetate succinate (HPMCAS), another enteric polymer, is also excluded when used alone.

Furthermore, it has been observed that the administration of bicalutamide in single agent therapy to humans causes an increase in the amount of testosterone circulating in the blood. Blackledge *et al,* (Urology, 1996, 47, Suppl. 1A), pp 44-47) discloses an approximate doubling of the basal level of total testosterone. It is believed that such an increase in the level of testosterone occurs when sufficient of the anti-androgen gains access to the CNS and blocks androgen receptors in the hypothalamus. The consequential lack of feedback of androgen causes additional release of LHRH by the hypothalamus which in turn causes release of luteinising hormone (LH) and follicle stimulating hormone (FSH) by the pituitary gland and production of testosterone in the testes. Aromatase enzyme in fat and other tissues converts some of the increased concentration of testosterone to oestradiol, which results in increased concentrations of oestrogen in the blood. Further discussion of this is provided by C Mahler *et al,* Clinical Pharmacokinetics, 1998, 34 (5), pp 405-417.

A disadvantageous effect is produced. Namely, the increase in the levels of circulating oestrogen may cause one or more of the side effects of gynaecomastia, breast tenderness, hot flushes, impotence and reduction in libido. A discussion on gynaecomastia can be found in C J Tyrrell, Prostate Cancer and Prostatic Diseases, 1999, 2(4): pp 167-171.

It would be further desirable to provide a pharmaceutical product or formulation comprising 4'-cyano-α',α',α'-trifluoro-3-(4-fluorophenylsulphonyl)-2-hydroxy-2-methylpropiono-*m*-toluidide with a reduction, relative to conventional bicalutamide therapy, of at least one side effect selected from gynaecomastia, breast tenderness, hot flushes, impotence and reduction in libido, preferably, while also fulfilling at least one of the following aims.

One aim is to improve upon the conventional formulation of bicalutamide (racemic 4'-cyano-α',α',α'-trifluoro-3-(4-fluorophenylsulphonyl)-2-hydroxy-2-methylpropiono-*m*-toluidide) by increasing the therapeutic potential of bicalutamide.

Another aim is to provide a 4'-cyano-α',α',α'-trifluoro-3-(4-fluorophenylsulphonyl)-2-hydroxy-2-methylpropiono-*m*-toluidide formulation having enhanced storage stability.

## SUMMARY OF THE INVENTION

The present invention fulfils this aim by providing a pharmaceutical product for mucosal administration to a patient, the product comprising 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in solid dispersion with an enteric polymer having a $pK_a$ from 3 to 6, the product further comprising an anti-oestrogen and/or an aromatase inhibitor. In one embodiment, wherein >50% of the 4'-cyano-α',α',α'-trifluoro-3-(4-fluorophenylsulphonyl)-2-hydroxy-2-methylpropiono-m-toluidide is provided in the form of the R-enantiomer. It is contemplated that one or a mixture of such enteric polymers can be used.

The invention also provides a pharmaceutical dose of 4'-cyano-α',α',α'-trifluoro-3-(4-fluorophenylsulphonyl)-2-hydroxy-2-methylpropiono-*m*-toluidide administrable to a patient for treating and/or reducing the risk of prostate cancer in the patient, wherein the dose comprises from 25 to 600 mg of 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in a solid dispersion with an enteric polymer having a $pK_a$ from 3 to 6, the dose further comprising an anti-oestrogen or an aromatase inhibitor. In one embodiment, wherein >50% of the 4'-cyano-α',α',α'-trifluoro-3-(4-fluorophenylsulphonyl)-2-hydroxy-2-methylpropiono-*m*-toluidide is provided in the form of the R-enantiomer. In a further embodiment the dose range is from 10 to 1000mg of bicalutamide.

Further aspects of the invention relate to the use in the manufacture of a pharmaceutical product of an anti-oestrogen or an aromatase inhibitor and 4'-cyano-α',α',α'-trifluoro-3-(4-fluorophenylsulphonyl)-2-hydroxy-2-methylpropiono-*m*-toluidide or a pharmaceutically acceptable salt or solvate thereof, for simultaneous or sequential administration to a patient, for treating and/or reducing the risk of prostate cancer in the patient and treating and/or preventing at least one side effect selected from gynaecomastia, breast tenderness, hot flushes, impotence and reduction in libido, the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6, and optionally wherein >50% of the the 4'-cyano-α',α',α'-trifluoro-3-(4-fluorophenylsulphonyl)-2-hydroxy-2-methylpropiono-*m*-toluidide is provided in the form of the R-enantiomer. **FIGURES**

Fig. 1 Dissolution of bicalutamide (ie, racemic 4'-cyano-α',α',α'-trifluoro-3-(4-fluorophenylsulphonyl)-2-hydroxy-2-methylpropiono-*m*-toluidide) from various solid dispersion formulations comprising enteric polymers (50mg bicalutamide in 900ml of media).

Key:-

| | |
|---|---|
| Circles | - conventional bicalutamide tablet formulation |
| Broken line | - HPMCP HP-55S |
| Diamonds | - EUDRAGIT™ L100 |
| Squares | - HPMCAS AQOAT™ LG |

Fig. 2 Dissolution of bicalutamide from various solid dispersion formulations comprising enteric or non-enteric polymers (50mg bicalutamide in 900ml of media).

Key:-

| | |
|---|---|
| Diamonds | - HPMC PHARMACOAT™606 |
| Squares | - METOLOSE™ 60SH 50cp |
| Triangles | - PEG4000 |
| Crosses | - PLA:PEG [2kDa:2kDa] |
| Broken line | - HPMCP HP-55S |
| Circles | - conventional bicalutamide tablet formulation |

Fig. 3 Dissolution of bicalutamide from solid dispersion formulations (50mg bicalutamide in 900ml of media) comprising bicalutamide with HP-55S at various weight ratios.

Key:-

The following ratios relate to weight ratios ofbicalutamide:HP-55S

| | |
|---|---|
| Diamonds | - 1:5 |
| Squares | - 1:4 |

(continued)

Key:-

| | |
|---|---|
| Triangles | - 1:3 |
| Crosses | - 1:2 |
| Circles | - 1:1 |
| Broken line | - conventional bicalutamide tablet formulation. |

Fig. 4 Plasma profiles following administration of bicalutamide formulations to dogs (n=6, 450mg bicalutamide dose). The vertical bars indicate variability.

Key: -

| | |
|---|---|
| Solid line | - solid dispersion of 1:3 by weight of bicalutamide:HP-55S |
| Broken line | - conventional bicalutamide tablet formulation. |

Fig. 5 Dissolution of bicalutamide (ie, racemic 4'-cyano-α',α',α'-trifluoro-3-(4-fluorophenylsulphonyl)-2-hydroxy-2-methylpropiono-*m*-toluidide) and optically pure R-4'-cyano-α',α',α'-trifluoro-3-(4-fluorophenylsulphonyl)-2-hydroxy-2-methylpropiono-*m*-toluidide) from solid dispersion formulations (50mg 4'-cyano-α',α',α'-trifluoro-3-(4-fluorophenylsulphonyl)-2-hydroxy-2-methylpropiono-*m*-toluidide in 900ml of media, 1:3 drug : polymer ratio).

Key:-

| | |
|---|---|
| Triangles | - conventional bicalutamide tablet formulation |
| Diamonds | - bicalutamide solid dispersion |
| Squares | - R-4'-cyano-α',α',α'-trifluoro-3-(4-fluorophenylsulphonyl)-2-hydroxy-2-methylpropiono-*m*-toluidide solid dispersion |

Fig. 6 Dissolution of bicalutamide (ie, racemic 4'-cyano-α',α',α'-trifluoro-3-(4-fluorophenylsulphonyl)-2-hydroxy-2-methylpropiono-*m*-toluidide) and optically pure R-4'-cyano-α',α',α'-trifluoro-3-(4-fluorophenylsulphonyl)-2-hydroxy-2-methylpropiono-*m*-toluidide) from solid dispersion formulations (50mg 4'-cyano-α',α',α'-trifluoro-3-(4-fluorophenylsulphonyl)-2-hydroxy-2-methylpropiono-*m*-toluidide in 900ml of media, 1:1 drug: polymer ratio).

Key:-

| | |
|---|---|
| Triangles | - conventional bicalutamide tablet formulation |
| Diamonds | - bicalutamide solid dispersion |
| Squares | - R-4'-cyano-α',α',α'-trifluoro-3-(4-fluorophenylsulphonyl)-2-hydroxy-2-methylpropiono-*m*-toluidide solid dispersion |

Fig.7 Combination formulation bicalutamide/tamoxifen dose ratio 150mg/10mg - pH shift dissolution; cumulative % released of bicalutamide with time

Key: -

| | |
|---|---|
| Diamonds | - bicalutamide:tamoxifen solid dispersion with HP-55S; drug:polymer ratio 1:3. |
| Squares | - 1:3 bicalutamide/HP-55S solid dispersion blended with tamoxifen |

Fig. 8 Combination formulation bicalutamide/tamoxifen dose ratio 150mg/10mg pH shift dissolution; cumulative % released of tamoxifen with time.

Key: -

| | |
|---|---|
| Diamonds | - bicalutamide:tamoxifen solid dispersion with HP-55S; drug:polymer ratio 1:3. |
| Squares | - 1:3 bicalutamide/HP-55S solid dispersion blended with tamoxifen |
| Triangles | - tamoxifen citrate |

Fig. 9 XRPD analysis of bicalutamide/tamoxifen/HP55s solid dispersion (A), tamoxifen blended with bicalutamide/HP-55s solid dispersion (B) and tamoxifen citrate only (C) samples to determine crystallinity content.

## DETAILED DESCRIPTION OF THE INVENTION

The inventors chose to investigate solid dispersion formulations as a possible means of fulfilling at least one of the aims stated above.

The prior art teaches a very wide range of possible polymers for solid dispersion, in order to increase the bioavailability of drugs in general. The inventors have now surprisingly found that the therapeutic potential of 4'-cyano-α',α',α'-trifluoro-3-(4-fluorophenylsulphonyl)-2-hydroxy-2-methylpropiono-*m*-toluidide can be increased by formulating 4'-cyano-α',α',α'-trifluoro-3-(4-fluorophenylsulphonyl)-2-hydroxy-2-methylpropiono-*m*-toluidide in a solid dispersion specifically with an enteric polymer having a $pK_a$ from 3 to 6. As the non-limiting example section below demonstrates, such an increase in therapeutic potential for 4'-cyano-α',α',α'-trifluoro-3-(4-fluorophenylsulphonyl)-2-hydroxy-2-methylpropiono-*m*-toluidide is not achieved with other polymers.

Various materials have conventionally been used to coat pharmaceutical tablets, capsules and granules to be compressed into tablets or used to fill capsules. Reference is made to Schroeter, L C, Coating of Tablets, Capsules and Pills, Remington's Pharmaceutical Sciences, $13^{th}$ ed., 1965, p. 604, which reviews more than 60 enteric coating materials. These include coating materials (eg, carnauba wax, stearic acid and paraffin) that rely on erosion in the intestinal tract, and enteric polymers that are designed to resist the destructive action of gastric fluid and to disintegrate in the intestinal tract. Enteric polymers are thus by definition pH-sensitive and have ionisable acid groups. The acid groups are nonionized and therefore poorly soluble in water. Ionisation, and thus increased solubility, occurs in the intestinal tract, so that the polymers are substantially insoluble in the low pH environment of the gastric fluid (pH 1 to 3.5), but rapidly dissolve at the pH of intestinal fluid, so that, as the dosage form empties into the duodenum a dramatic change in pH occurs, leading to ionisation of the acid groups and increased solubility. The particular enteric polymers used in the present invention are those enteric polymers that have a $pK_a$ from 3 to 6. In one example, the lower end of this range is 3.5, 4 or 4.5. In one example, the upper end of the range is 5 or 5.5.

As the skilled addressee knows, the Henderson-Hasselbach equation may be used to determine the $pK_a$ according to the following equation:-

$$pK_a = pH - \log\,(\text{concentration of non-ionised polymer} \div \text{concentration of ionised polymer})$$

At a pH two units below the $pK_a$, only approximately 1% of the acid groups will be ionised, and the polymer will be poorly soluble in gastric fluid. As the pH increases, the percentage of ionised acid groups increases, such that when the pH exceeds the $pK_a$ by two units the percentage of ionised groups is approximately 100%, and the polymer will be soluble in the intestines.

In one embodiment, the enteric polymer is selected from hydroxypropyl methylcellulose acetate succinate (HPMCAS), hydroxpropyl methylcellulose acetate pthalate, hydroxypropyl methylcellulose acetate, hydroxypropyl methylcellulose succinate, a methacrylic acid copolymer, polyvinyl acetate phthalate (PVAP), cellulose acetate phthalate (CAP), methylcellulose acetate phthalate, ethyl cellulose acetate phthalate, hydroxypropyl cellulose acetate phthalate, hydroxypropyl methylcellulose phthalate (HPMCP), cellulose proprionate pthalate, hydroxypropyl cellulose butyrate pthalate, hydroxypropyl cellulose acetate pthalate succinate, hydroxypropyl methylcellulose trimellitate, cellulose acetate trimellitate (CAT), methylcellulose acetate trimellitate, ethyl cellulose acetate trimellitate, hydroxypropyl cellulose acetate trimellitate, hydroxypropyl methylcellulose acetate trimellitate, hydroxypropyl cellulose acetate trimellitate succinate, cellulose proprionate trimellitate, cellulose butyrate trimellitate, cellulose acetate terepthalate and cellulose acetate isopthalate.

The use of the term "hydroxypropyl methylcellulose phthalate polymer", or HPMCP, is known to the skilled reader for classifying a group of polymers which share the same basic structural features and include such polymers as: hypromellose phthalate; methylhydroxypropylcellulosi pthalas; cellulose, hydrogen 1,2-benzenedicarboxylate, 2-hydroxypropyl methyl; as well as commercially available polymers HP-55™, HP-55S™ and HP-50™ (available from Shin-Etsu Chemical Industry Co., Ltd., Japan or appointed distributors).

Preferably the hydroxypropylmethylcellulose phthalate polymer has a molecular weight (Mw) from 20kDa to 200kDa, eg from 80kDa to 130kDa. In one embodiment, the Mw is less than 150kDa, or less than 100kDa. HP-50, HP-55 and HP-55S are examples of hydroxypropylmethylcellulose phthalate polymers. HP-55 has a Mw 84kDa. HP-55S has a Mw of 132kDa. HP-50 has a Mw 78kDa. HP-50 is soluble at pH≥5, whereas HP-55 and HP-55S are soluble at pH≥5.5. In one embodiment, the bicalutamide is in a solid dispersion with at least one polymer selected from HP-50, HP-55 and HP-55S. Thus, it is contemplated that a mixture of two or more of these HPMCP polymers can be used.

HPMCAS (trade name: AQOAT, available from Shin-Etsu Chemical Industry Co., Ltd., Japan or appointed distributors) is available in the following grades: AS-LF, AS-MF, AS-HF, AS-LG, AS-MG and AS-HG. The AS-L grades are soluble at pH ≥5.5, the AS-M grades are soluble at pH≥ 6.0 and the AS-H grades are soluble at pH≥ 6.5. In one embodiment, the bicalutamide is in a solid dispersion with at least one polymer selected from HPMCAS grades AS-L, AS-M, AS-H. Thus, it is contemplated that a mixture of two or more of these HPMCAS polymers can be used.

Methacrylic acid copolymer is a fully polymerised copolymer of methacrylic acid and methacrylic acid methyl ester. Grade A (trade name: EUDRAGIT™ L 100, available from Rohm Pharma or appointed distributors) and grade B (trade name EUDRAGIT™ S 100) are available. The grades differ in the ratio of free carboxyl groups to ester groups and, therefore, differ in solubility profiles. Type A has a ratio of approximately 1:1 and is soluble at pH≥6. Type B has a ratio of approximately 1:2 and is soluble at pH≥7. Another grade (EUDRAGIT™ L 30 D-55) is soluble at pH≥5.5. In one embodiment, the bicalutamide is in a solid dispersion with at least one methacrylic acid copolymer. Thus, it is contemplated that a mixture of two or more of these polymers (eg, grades A and B) can be used.

PVAP is soluble at pH≥5 and is available from Colorcon Inc or appointed distributors.

CAP (available from FMC Corporation as part of a powdered product, AQUATERIC™) solubilises at pH≥6.5.

CAT is available from Eastman Fine chemicals, Zurich, Switzerland.

The term "solid dispersion" is a well-known term in the art, which refers to a dispersion of one or more active ingredients in an inert carrier or matrix at solid state, typically, but not exclusively, prepared by conventional melting (fusion), solvent, or melting-solvent methods. Terms also used to describe this type of approach are solid solutions, coevaporates and coprecipitates (W.L. Chiou and S. Riegelman, "Applications of Solid Dispersion Systems", J. Pharm. Sci. 60:1281-1302, 1971). In one embodiment the dispersion is manufactured by melt extrusion.

A preferred ratio of 4'-cyano-α',α',α'-trifluoro-3-(4-fluorophenylsulphonyl)-2-hydroxy-2-methylpropiono-*m*-toluidide: enteric polymer by weight is from 1:0.25 to 1:10. More preferably the lower limit of this range is 1:0.5, 1:0.75 or 1: 1. Preferably, the upper limit of this range is 1:<3, 1:3 or 1:5. Examples of ranges of ratios are 1:1 to 1:3 or 1:0.25 to 1:<3.

One aspect of the invention provides a daily pharmaceutical dose of 25 to 600mg of 4'-cyano-α',α',α'-trifluoro-3-(4-fluorophenylsulphonyl)-2-hydroxy-2-methylpropiono-*m*-toluidide administrable to a patient for treating and/or reducing the risk of prostate cancer in the patient, wherein the dose comprises 4'-cyano-α',α',α'-trifluoro-3-(4-fluorophenylsulphonyl)-2-hydroxy-2-methylpropiono-*m*-toluidide in a solid dispersion comprising an enteric polymer having a $pK_a$ from 3 to 6, and, optionally, wherein >50% of the 4'-cyano-α',α',α'-trifluoro-3-(4-fluorophenylsulphonyl)-2-hydroxy-2-methylpropiono-*m*-toluidide is provided in the form of the R-enantiomer. Preferably, the dose comprises an upper limit of 1000, 500, 450, 400, 300, 200, 150, 125, 100, 75 , 50mg or 25mg of 4'-cyano-α',α',α'-trifluoro-3-(4-fluorophenylsulphonyl)-2-hydroxy-2-methylpropiono-*m*-toluidide. In one example, the dose comprises 450mg of 4'-cyano-α',α',α'-trifluoro-3-(4-fluorophenylsulphonyl)-2-hydroxy-2-methylpropiono-*m*-toluidide. The daily, once a day, dose is preferably provided in a single unit format, e.g. tablet or capsule. However, multiple dose units (e.g. 1, 2, 3 etc.) are also encompassed.

Additional excipients may be included in the formulation or dose. For example, the formulation or dose may comprise one or more fillers, binder, disintegrants and/or lubricants.

Suitable fillers include, for example, lactose, sugar, starches, modified starches, mannitol, sorbitol, inorganic salts, cellulose derivatives (e.g. microcrystalline cellulose, cellulose), calcium sulphate, xylitol and lactitol.

Suitable binders include, for example, polyvinylpyrrolidone, lactose, starches, modified starches, sugars, gum acacia, gum tragacanth, guar gum, pectin, wax binders, microcrystalline cellulose, methylcellulose, carboxymethylcellulose, hydroxypropyl methylcellulose, hydroxyethyl cellulose, hydroxypropyl cellulose, copolyvidone, gelatin and sodium alginate.

Suitable disintegrants include, for example, crosscarmellose sodium, crospovidone, polyvinylpyrrolidone, sodium starch glycollate, corn starch, microcrystalline cellulose, hydroxypropyl methylcellulose and hydroxypropyl cellulose.

Suitable lubricants include, for example, magnesium stearate, stearic acid, palmitic acid, calcium stearate, talc, carnauba wax, hydrogenated vegetable oils, mineral oil, polyethylene glycols and sodium stearyl fumarate.

Additional conventional excipients which may be added include preservatives, stabilisers, anti-oxidants, silica flow conditioners, antiadherents or glidants.

Other suitable fillers, binders, disintegrants, lubricants and additional excipients which may be used are described in the Handbook of Pharmaceutical Excipients, 3rd Edition; The Theory and Practice of Industrial Pharmacy, 3rd Edition 1986; Pharmaceutical Dosage Forms 1998; Modern Pharmaceutics, 3rd Edition 1995; Remington's Pharmaceutical Sciences 20th Edition 2000.

Preferably, the 4'-cyano-α',α',α'-trifluoro-3-(4-fluorophenylsulphonyl)-2-hydroxy-2-methylpropiono-*m*-toluidide will be present in an amount of 1 to 80% , and preferably from 1 to 50% (more preferably 2 to 25% or 2 to 15%) by weight of the solid dispersion.

Preferably, one or more fillers will be present in an amount of 1 to 70% by weight of the formulation or dose.

Preferably, one or more binders will be present in an amount of 2 to 40% by weight of the formulation or dose.

Preferably, one or more disintegrants will be present in an amount of 0.5 to 25%, and especially 4 to 10% by

weight of the formulation or dose.

It will be appreciated that a particular excipient may act as both a binder and a filler, or as a binder, a filler and a disintegrant. Typically the combined amount of filler, binder and disintegrant comprises, for example, 1 to 90% by weight of the formulation or dose.

Preferably, one or more lubricants will be present in an amount of 0.25 to 5%, and especially 1 to 2% by weight of the formulation or dose.

Preferably, one or more wetting agents will be present in the solid dispersion in an amount of 0.1 to 5% (more preferably, 1 to 2%) by weight of the solid dispersion. The presence of a wetting agent provides a further enhancement of the increase in therapeutic potential achieved with the present invention. Examples of suitable wetting agents include sodium dodecyl sulphate (sodium lauryl sulphate); docusate sodium; polyoxyethylen sorbitan fatty acid esters, eg polysorbates 20, 40, 60 and 80; polyoxyethylene castor oil derivatives, eg Cremophor RH40™ ; and poloxamers.

Methods for preparing solid dispersions are known in the art and typically comprise the steps of dissolving the drug and the polymer in a common solvent and evaporating the solvent. The solvent can be routinely selected according to the polymer used and the preparation method. Examples of solvents are: acetone, acetone/dichloromethane, methanol/dichloromethane, acetone/water, acetone/ethanol, dichloromethane/ethanol or ethanol/water. For HP-50, for example, the last four solvents can be used. For HPMCAS, for example, acetone, methanol, ethanol/water and methylene chloride/ethanol can be used. For methacrylic acid copolymers, isopropyl alcohol can be used. For polyvninly acetate phthalate, for example, methanol, ethanol, acetone/methanol, acetone/ethanol and methanol/methylene chloride can be used. For CAP, for example, ether/alcohols, ketones (eg, acetone), esters and cyclic ethers can be used. Methods for evaporating solvent include rotary evaporation, spray drying, lyophilisation and thin film evaporation. Other techniques may be used such as solvent controlled precipitation, pH controlled precipitation, spray congealing, melt extrusion and supercritical fluid technology.

When referring to a solid dispersion we do not exclude the possibility that a proportion of the 4'-cyano-α',α',α'-trifluoro-3-(4-fluorophenylsulphonyl)-2-hydroxy-2-methylpropiono-*m*-toluidide may be dissolved within the polymer used, the exact proportion, if any, will depend upon the particular enteric polymer(s) selected.

In the formulations of the invention, at least some of the 4'-cyano-α',α',α'-trifluoro-3-(4-fluorophenylsulphonyl)-2-hydroxy-2-methylpropiono-*m*-toluidide may be present in amorphous form in the solid dispersion with the enteric polymer. The provision of the 4'-cyano-α',α',α'-trifluoro-3-(4-fluorophenylsulphonyl)-2-hydroxy-2-methylpropiono-*m*-toluidide in amorphous form is additionally advantageous, since it further increases the solubility and dissolution rate of the 4'-cyano-α',α',α'-trifluoro-3-(4-fluorophenylsulphonyl)-2-hydroxy-2-methylpropiono-*m*-toluidide, thereby enhancing the increase in therapeutic potential achieved with the present invention. Whether or not drug is present in amorphous form can be determined by conventional thermal analysis or X-ray diffraction. In one embodiment, at least 25% of the 4'-cyano-α',α',α'-trifluoro-3-(4-fluorophenylsulphonyl)-2-hydroxy-2-methylpropiono-*m*-toluidide in the formulation is present in amorphous form. More preferably, this amount is at least 30%, 40%, 50%, 75%, 90%, 95% or 99%. The most preferred embodiment is where 100% of the 4'-cyano-α',α',α'-trifluoro-3-(4-fluorophenylsulphonyl)-2-hydroxy-2-methylpropiono-*m*-toluidide in the formulation is in amorphous form. The amorphous form applies to the bicalutamide drug as a whole, thus the proportion of amorphous drug can be S-enantiomer or R-enantiomer or both.

The formulations and doses are preferably mucosally administrable, ie administrable to mucosal membranes for absorption across the membranes. To this end, suitable routes of administration include administration by inhalation, as well as oral, intranasal and rectal administration. Oral administration is particularly preferred. A tablet or other form of the formulation would be chosen by the skilled addressee according to the route of administration.

The 4'-cyano-α',α',α'-trifluoro-3-(4-fluorophenylsulphonyl)-2-hydroxy-2-methylpropiono-*m*-toluidide is useful to provide an anti-androgenic effect, in that this compound blocks androgen activity in a patient. The anti-androgenic effect is useful for treating cancer, for example prostate cancer. Particular examples are advanced prostate cancer and early prostate cancer. The anti-androgenic effect may be useful for prophylaxis, in order to reduce the risk of prostate cancer occurrence in patients or re-occurrence (eg, following prostatectomy or radiation therapy aimed at curing the patient). This could be especially useful in men genetically pre-disposed to prostate cancer. Conventional methods are available to classify patients according to their risk of contracting prostate cancer, for example by assessment of family history and measurements over time of particular blood proteins such as prostate specific antigen (PSA). Other uses for the anti-androgenic effect are the treatment of a non-malignant disease of the prostate gland (eg, benign prostatic hyperplasia or hypertrophy), testotoxicosis, hirsutism and acne. These conditions, in conjunction with prostate cancer, will be referred to herein as prostatic disorders.

The patient can be a human male, eg an adult, but the treatment of other mammals is also contemplated.

In one embodiment of the formulation or dose, >50%, ≥60%, ≥70%, ≥80%, ≥85%, ≥90%, ≥95%, ≥98% or ≥99% or thereabout of the 4'-cyano-α',α',α'-trifluoro-3-(4-fluorophenylsulphonyl)-2-hydroxy-2-methylpropiono-*m*-toluidide is provided in the form of the R-enantiomer. In a preferred embodiment, 100% or substantially 100% of the 4'-cyano-α',α',α'-trifluoro-3-(4-fluorophenylsulphonyl)-2-hydroxy-2-methylpropiono-*m*-toluidide is provided in the form of the R-enantiomer. By "substantially 100%" we mean that the 4'-cyano-α',α',α'-trifluoro-3-(4-fluorophenylsulphonyl)-2-hydroxy-2-methylpropiono-*m*-toluidide is provided as the pure R-enantiomer, or there is a trace (<1%) of the S-enantiomer present. As the experimental section below shows, the predominance of the R-enantiomer in the present invention provides for a 4'-cyano-α',α',α'-trifluoro-3-(4-fluorophenylsulphonyl)-2-hydroxy-2-methylpropiono-*m*-toluidide formulation with good storage stability and an enhanced therapeutic potential.

To fulfil the aim of providing a pharmaceutical product comprising bicalutamide with reduced side effects relative to conventional bicalutamide pharmaceutical product, the present invention provides a pharmaceutical product for administration to a patient, the formulation comprising 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in a solid dispersion comprising an enteric polymer having a $pK_a$ from 3 to 6, the formulation further comprising an anti-oestrogen (eg, tamoxifen or a pharmaceutically acceptable salt or solvate thereof, eg, tamoxifen citrate). It is contemplated that one or a mixture of such enteric polymers can be used. Further details of suitable solid dispersions is given in the general description above (with the exception that the present aspect is not limited to the use of a drug proportion of >50% of the 4'-cyano-α',α',α'-trifluoro-3-(4-fluorophenylsulphonyl)-2-hydroxy-2-methylpropiono-*m*-toluidide being provided in the form of the R-enantiomer).

The anti-oestrogen prevents oestrogen activity. The anti-oestrogenic effect is useful for treating and/or preventing a side effect selected from gynaecomastia, breast tenderness, hot flushes, impotence, reduction in libido, nausea, vomiting, fatigue and diarrhoea. Such side effects have been observed with monotherapy use of anti-androgens. Preferably, the side effect is one or both of gynaecomastia and breast tenderness.

Tamoxifen, an anti-oestrogen, is known by the AstraZeneca trade name NOLVADEX™. Tamoxifen is the trans isomer of 1-(*p*-beta-dimethylaminoethoxyphenyl)-1,2-diphenylbut-1-ene, which is disclosed in US-4,536,516. An alternative name is (Z)-2-[*p*-(1,2-diphenylbut-1-enyl)phenoxy]ethyldimethylamine. The corresponding structure is shown in formula I:-

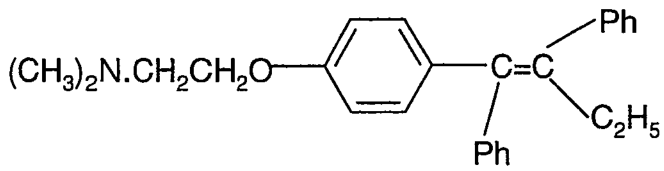

I

Preferably, the 4'-cyano-α',α',α'-trifluoro-3-(4-fluorophenylsulphonyl)-2-hydroxy-2-methylpropiono-*m*-toluidide and anti-oestrogen are provided in a ratio respectively of 25 to 350 (preferably the lower end of the range being 50; preferably the upper end of the range being 300, 150 or 50; suitable values in the ranges being 150 or 50) : 0.5 to 100 (preferably lower end of the range being 1, 2.5 or 5; preferably the upper end of the range being 40, 20 or 10; a suitable value in the range being 2.5, 5, 7.5, 8, 9, 10, 15 or 20).

The invention also provides a pharmaceutical product for mucosal administration to a patient, the formulation comprising 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in solid dispersion comprising an enteric polymer having a $pK_a$ from 3 to 6, the formulation further comprising an aromatase inhibitor (eg, anastrozole, letrozole or exemestane, or a pharmaceutically acceptable salt or solvate thereof). It is contemplated that one or a mixture of such enteric polymers can be used. Further details of suitable solid dispersions is given in the general description above. In one embodiment, >50% of the 4'-cyano-α',α',α'-trifluoro-3-(4-fluorophenylsulphonyl)-2-hydroxy-2-methylpropiono-*m*-toluidide is provided in the form of the R-enantiomer.

The aromatase inhibitor inhibits conversion of testosterone to oestradiol by aromatase enzyme. The aromatase inhibition is useful for treating and/or preventing a side effect selected from gynaecomastia, breast tenderness, hot flushes, impotence, reduction in libido, nausea, vomiting, fatigue and diarrhoea. Such side effects have been observed with monotherapy use of anti-androgens. Preferably, the side effect is one or both of gynaecomastia and breast tenderness.

Anastrozole, an aromatase inhibitor, is known by the AstraZeneca trade name ARIMIDEX™. Anastrozole is known as 2,2'-[5-(1*H*-1,2,4-triazol-1-ylmethyl)-1,3-phenylene]di(2-methylpropionitrile), which is disclosed in US re-issue No. 36,617. An alternative name is 2,2'-dimethyl-2,2'-[5-(1*H*-1,2,4-triazol-1-ylmethyl)-1,3-phenylene]bis(propiononitrile). The corresponding structure is shown in formula II:-

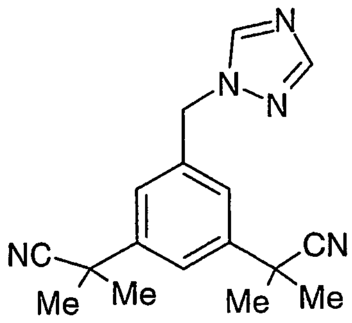

II

Letrozole, an aromatase inhibitor, is known by the trade name FEMARA™. Letrozole is known by the alternative names 4,4'-(1*H*-1,2,4-triazol-1-ylmethylene)-bisbenzonitrile; 1-[bis(4-cyanophenyl)methyl]-1,2,4-triazole; and 4-[1-(4-cyanophenyl)-1-(1,2,4-triazol-1-yl)methyl]benzonitrile. Letrozole is disclosed in US 4,978,672. The corresponding structure is shown in formula III:-

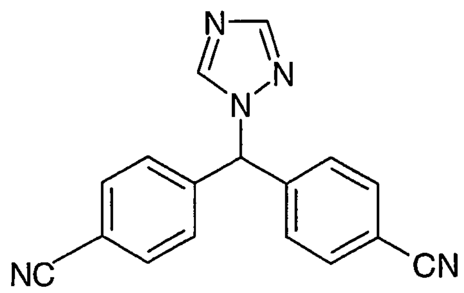

III

Exemestane, an aromatase inhibitor, is known by the trade name AROMASIN™ and is marketed by Pharmacia and Upjohn. Exemestane is known by the alternative name 6-methylenandrosta-1,4-diene-3,17-dione. Further reference is made to US-4,808,616 and US-4,904,650.

Preferably, the 4'-cyano-α',α',α'-trifluoro-3-(4-fluorophenylsulphonyl)-2-hydroxy-2-methylpropiono-*m*-toluidide and aromatase inhibitor are provided in a ratio respectively of 25 to 350 (preferably the lower end of the range being 50; preferably the upper end of the range being 300, 150 or 50; suitable values in the ranges being 150, 80 or 50) : 0.005 to 100 (preferably the lower end of the range being 0.05 or 0.5; preferably the upper end of the range being 50, 10 or 1; the most preferred range being 0.5 to 1; a suitable value in the range being 1).

The invention also provides a pharmaceutical dose of 25 to 600 mg of 4'-cyano-α',α',α'-trifluoro-3-(4-fluorophenylsulphonyl)-2-hydroxy-2-methylpropiono-*m*-toluidide administrable to a patient for treating and/or reducing the risk of prostate cancer in the patient, wherein the dose comprises 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in a solid dispersion comprising an enteric polymer having a $pK_a$ from 3 to 6, the dose further comprising an anti-oestrogen.

A suitable pharmaceutical dose has from 0.5 to 200 mg of the anti-oestrogen. Preferably, the lower end of the range is 1, 5, 10, 15 or 20 mg; preferably the upper end of the range is 80, 60, 40, 20 or 10 mg; a suitable value in the range being 10 or 20 mg. The dose or the regimen has from 25 to 600 mg of the compound 4'-cyano-α',α',α'-trifluoro-3-(4-fluorophenylsulphonyl)-2-hydroxy-2-methylpropiono-*m*-toluidide or a pharmaceutically acceptable salt or solvate thereof. Preferably the lower end of the range is 25 mg; preferably the upper end of the range is 300, 150 or 50 mg;

suitable values in the ranges are 150 or 50 mg. In one example, the dose is 150 mg of 4'-cyano-α',α',α'-trifluoro-3-(4-fluorophenylsulphonyl)-2-hydroxy-2-methylpropiono-*m*-toluidide or a pharmaceutically acceptable salt or solvate thereof and 1, 2.5, 5, 7.5, 8, 9, 10, 15 or 20 mg of the anti-oestrogen (eg, tamoxifen citrate).

In addition, the invention provides a pharmaceutical dose of 25 to 600 mg of 4'-cyano-α',α',α'-trifluoro-3-(4-fluorophenylsulphonyl)-2-hydroxy-2-methylpropiono-*m*-toluidide administrable to a patient for treating and/or reducing the risk of prostate cancer in the patient, wherein the dose comprises 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in a solid dispersion comprising an enteric polymer having a $pK_a$ from 3 to 6, the dose further comprising an aromatase inhibitor.

A suitable pharmaceutical dose has from 0.005 to 200 mg of the aromatase inhibitor. Preferably, the lower end of the range is 0.05 or 0.5 mg; preferably the upper end of the range is 50, 10 or 1 mg; the most preferred range is 0.5 to 1 mg; a suitable value in the range being 1 mg. The dose or the regimen has from 25 to 600 mg of the compound 4'-cyano-α',α',α'-trifluoro-3-(4-fluorophenylsulphonyl)-2-hydroxy-2-methylpropiono-m-toluidide or a pharmaceutically acceptable salt or solvate thereof. Preferably the lower end of the range is 25 mg; preferably the upper end of the range is 300, 150 or 50 mg; suitable values in the ranges are 150 or 50 mg. In one example, the dose is 150 mg of 4'-cyano-α',α',α'-trifluoro-3-(4-fluorophenylsulphonyl)-2-hydroxy-2-methylpropiono-*m*-toluidide or a pharmaceutically acceptable salt or solvate thereof and 0.1, 0.25, 0.5 or 1 mg of the aromatase inhibitor (eg, anastrozole).

The anti-oestrogen/aromatase inhibitor and the 4'-cyano-α',α',α'-trifluoro-3-(4-fluorophenylsulphonyl)-2-hydroxy-2-methylpropiono-*m*-toluidide are preferably administered daily. Another possible regime would be dosing of the 4'-cyano-α',α',α'-trifluoro-3-(4-fluorophenylsulphonyl)-2-hydroxy-2-methylpropiono-*m*-toluidide on alternate days and dosing of the anti-oestrogen/aromatase also on (the same or different) alternate days. To this end, the pharmaceutical product may include administration instructions. Preferably, the 4'-cyano-α',α',α'-trifluoro-3-(4-fluorophenylsulphonyl)-2-hydroxy-2-methylpropiono-*m*-toluidide is administered every 3, 4, 5, 6 or 7 days and the anti-oestrogen/aromatase is administered every 3, 4, 5, 6 or 7 days (eg, on the same day as the 4'-cyano-α',α',α'-trifluoro-3-(4-fluorophenylsulphonyl)-2-hydroxy-2-methylpropiono-*m*-toluidide).

Further aspects of the invention relate to the use in the manufacture of a pharmaceutical product of an anti-oestrogen and 4'-cyano-α',α',α'-trifluoro-3-(4-fluorophenylsulphonyl)-2-hydroxy-2-methylpropiono-*m*-toluidide or a pharmaceutically acceptable salt or solvate thereof, for simultaneous or sequential administration to a patient, for treating and/or preventing at least one side effect selected from gynaecomastia, breast tenderness, hot flushes, impotence and reduction in libido, and

(a) increasing the bioavailability of 4'-cyano-α',α',α'-trifluoro-3-(4-fluorophenylsulphonyl)-2-hydroxy-2-methylpropiono-*m*-toluidide in the patient; or
(b) treating and/or reducing the risk of prostate cancer in the patient. As explained below, reducing the risk of prostate cancer includes reducing the risk of re-occurrence of prostate cancer,

the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6.

The patient can be a human male, eg an adult, but the treatment of other mammals (except rats) is also contemplated.

By "treating" the side effect(s), we mean reducing the severity of a side effect or eliminating a side effect already being experienced by a patient. By "preventing" the side effect(s), we mean suppressing increase in the incidence or severity of a side effect.

In addition, the invention relates to the use in the manufacture of a pharmaceutical product of an anti-oestrogen and 4'-cyano-α',α',α'-trifluoro-3-(4-fluorophenylsulphonyl)-2-hydroxy-2-methylpropiono-*m*-toluidide or a pharmaceutically acceptable salt or solvate thereof, for simultaneous or sequential administration to patients, for reducing inter-patient variability in plasma concentrations of 4'-cyano-α',α',α'-trifluoro-3-(4-fluorophenylsulphonyl)-2-hydroxy-2-methylpropiono-*m*-toluidide in the patient and treating and/or preventing at least one side effect selected from gynaecomastia, breast tenderness, hot flushes, impotence and reduction in libido, the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6.

Furthermore, the invention relates to the use in the manufacture of a pharmaceutical product of an aromatase inhibitor and 4'-cyano-α',α',α'-trifluoro-3-(4-fluorophenylsulphonyl)-2-hydroxy-2-methylpropiono-*m*-toluidide or a pharmaceutically acceptable salt or solvate thereof, for simultaneous or sequential administration to a patient, for treating and/or preventing at least one side effect selected from gynaecomastia, breast tenderness, hot flushes, impotence and reduction in libido, and

(a) increasing the bioavailability of 4'-cyano-α',α',α'-trifluoro-3-(4-fluorophenylsulphonyl)-2-hydroxy-2-methylpropiono-*m*-toluidide in the patient; or
(b) treating and/or reducing the risk of prostate cancer in the patient. As explained below, reducing the risk of prostate

cancer includes reducing the risk of re-occurrence of prostate cancer,

the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6

In addition, the invention relates to the use in the manufacture of a pharmaceutical product of an aromatase inhibitor and 4'-cyano-α',α',α'-trifluoro-3-(4-fluorophenylsulphonyl)-2-hydroxy-2-methylpropiono-*m*-toluidide or a pharmaceutically acceptable salt or solvate thereof, for simultaneous or sequential administration to patients, for reducing inter-patient variability in plasma concentrations of 4'-cyano-α',α',α'-trifluoro-3-(4-fluorophenylsulphonyl)-2-hydroxy-2-methylpropiono-*m*-toluidide in the patient, relative to conventional bicalutamide pharmaceutical product, and treating and/or preventing at least one side effect selected from gynaecomastia, breast tenderness, hot flushes, impotence and reduction in libido, the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6.

The term "product" is intended to mean either a combination of the solid dispersion formulation and the anti-oestrogen/ aromatase inhibitor (eg, provided as a capsule or tablet containing both the solid dispersion and the anti-oestrogen/aromatase inhibitor) or a kit comprising separate amounts of the solid dispersion and the anti-oestrogen/ aromatase inhibitor (eg, a set of tamoxifen citrate tablets and a separate set of tablets of the solid dispersion). The latter product can be used for simultaneous or sequential (ie, temporally spaced) administration of the agents to the patient, while the combination is for simultaneous administration. Factors such as the rate of absorption, metabolism and the rate of excretion of each agent will affect their presence at the tumour site. Such factors are routinely considered by, and are well within the ordinary skill of, the clinician when he contemplates the treatment of a medical condition, which requires the conjoint administration of two agents in order to obtain a beneficial effect.

In one embodiment, the anti-oestrogen/aromatase inhibitor is provided in the solid dispersion, along with the 4'-cyano-α',α',α'-trifluoro-3-(4-fluorophenylsulphonyl)-2-hydroxy-2-methylpropiono-*m*-toluidide. Production of this embodiment entails the formation of a solution comprising the anti-oestrogen/aromatase inhibitor, the polymer and the 4'-cyano-α',α',α'-trifluoro-3-(4-fluorophenylsulphonyl)-2-hydroxy-2-methylpropiono-*m*-toluidide prior to spray drying (or other method described above for removing the solvent).

In another embodiment, the solid dispersion of the polymer and the 4'-cyano-α',α',α'-trifluoro-3-(4-fluorophenylsulphonyl)-2-hydroxy-2-methylpropiono-*m*-toluidide is produced and then mixed with the anti-oestrogen/aromatase inhibitor. Routine considerations of a person skilled in the art would be the particle size, particle size distribution, particle morphology and powder flow properties of the anti-oestrogen/aromatase inhibitor. The anti-oestrogen/aromatase inhibitor would be mixed with the solid dispersion using conventional mixing methods such as trituration or ordered mixing to attain the required content uniformity. Further details of these routine considerations is given in *Pharmaceutics, The science of dosage form design,* Edited by M E Aulton 1988.

As noted above, in one embodiment of the pharmaceutical product and doses according to the invention, >50% of the 4'-cyano-α',α',α'-trifluoro-3-(4-fluorophenylsulphonyl)-2-hydroxy-2-methylpropiono-*m*-toluidide is provided in the form of the R-enantiomer. In this embodiment, preferably about >50, ≥60%, ≥70%, ≥80%, ≥85%, ≥90%, ≥95%, ≥98% or ≥99%, or substantially 100% of the 4'-cyano-α',α',α'-trifluoro-3-(4-fluorophenylsulphonyl)-2-hydroxy-2-methylpropiono-*m*-toluidide is provided in the form of the R-enantiomer.

In another embodiment, the racemate of 4'-cyano-α',α',α'-trifluoro-3-(4-fluorophenylsulphonyl)-2-hydroxy-2-methylpropiono-*m*-toluidide is used.

According to another aspect of the invention there is provided a method for preparing a pharmaceutical formulation comprising 4'-cyano-α',α',α'-trifluoro-3-(4-fluorophenylsulphonyl)-2-hydroxy-2-methylpropiono-*m*-toluidide with reduced inter-patient variability in plasma concentrations of 4'-cyano-α',α',α'-trifluoro-3-(4-fluorophenylsulphonyl)-2-hydroxy-2-methylpropiono-*m*-toluidide and/or increased bioavailability of 4'-cyano-α',α',α'-trifluoro-3-(4-fluorophenylsulphonyl)-2-hydroxy-2-methylpropiono-*m*-toluidide and/or a reduced side effect selected from gynaecomastia, breast tenderness, hot flushes, impotence and reduction in libido, in the patient comprising forming a solid dispersion of an enteric polymer having a $pK_a$ from 3 to 6 with 4'-cyano-α',α',α'-trifluoro-3-(4-fluorophenylsulphonyl)-2-hydroxy-2-methylpropiono-*m*-toluidide, the formulation further comprising an antioestrogen. In one particular embodiment the antioestrogen is tamoxifen, in a further embodiment, greater than 50% of the 4'-cyano-α',α',α'-trifluoro-3-(4-fluorophenylsulphonyl)-2-hydroxy-2-methylpropiono-*m*-toluidide is provided in the form of the R-enantiomer. In a further embodiment greater that 30% of the 4'-cyano-α',α',α'-trifluoro-3-(4-fluorophenylsulphonyl)-2-hydroxy-2-methylpropiono-*m*-toluidide is in amorphous form.

Each of these aspects of increased bioavailability, enhanced storage stability and reduced interpatient variability are either relative to the same bioequivalent dose of conventional bicalutamide formulations.

The products and doses may be in a form suitable for oral use (for example as tablets, capsules, aqueous or oily suspensions, emulsions or dispersible powders or granules), for topical use (for example as creams, ointments, gels, or aqueous or oily solutions or suspensions; for example for use within a transdermal patch), for parenteral administration (for example as a sterile aqueous or oily solution or suspension for intravenous, subcutaneous, intramuscular or intravascular dosing), or as a suppository for rectal dosing. Preferably a form suitable for oral administration is used, for example as tablets or capsules.

The products and doses may also use conventional pharmaceutically-acceptable diluents or carriers that are well known in the art. Suitable pharmaceutically-acceptable diluents or carriers for a tablet formulation include, for example, inert diluents such as lactose, sodium carbonate, calcium phosphate or calcium carbonate, granulating and disintegrating agents such such as croscarmellose sodium, sodium starch glycollate,com starch or alginic acid; binding agents such as Polyvinylpyrrolidone, gelatin or starch; lubricating agents such as magnesium stearate, stearic acid or talc; preservative agents such as ethyl or propyl p-hydroxybenzoate, silica flow conditioners, antiadherents or glidants and anti-oxidants, such as ascorbic acid. Tablet formulations may be uncoated or coated either to modify their disintegration and the subsequent absorption of the active ingredient within the gastrointestinal tract, or to improve their stability and/or appearance, in either case using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin or olive oil.

## EXPERIMENTAL

### A: Comparative Examples

The following examples are not according to the present invention, but are included to provide a suitable context for the interpretation of the examples according to the present invention (see section B onwards).

### *In Vitro* Assessment of Various Solid Dispersion Formulations

The inventors formulated a solid dispersion of bicalutamide (racemic a 4'-cyano-α',α',α'-trifluoro-3-(4-fluorophenylsulphonyl)-2-hydroxy-2-methylpropiono-*m*-toluidide) with representative enteric polymers having a $pK_a$ in the range of 3 to 6 (in this case HPMCP HP-55S, EUDRAGIT L100 and HPMCAS AQOAT LG) and compared these against a conventional bicalutamide tablet formulation and also (using HPMCP HP-55S as a representative enteric polymer) against solid dispersions using several different non-enteric polymers (polyethylene glycol (PEG) 4000, PLA:PEG [2kDa: 2kDa] (polylactide:methoxypolyethylene glycol [2kDa:2kDa]), hydroxypropyl methylcellulose (HPMC) PHARMACOAT™ 606 and METOLOSE 60SH 50cp) with bicalutamide. Each formulation had a weight ratio of bicalutamide:polymer of 1: 5. The formulations were assessed for an improvement in therapeutic potential using an *in vitro* dissolution test.

The performance of solid dispersions having varying weight ratios of bicalutamide:HP-55S was also assessed.

#### Preparation of Solid Dispersion Formulations

Solid dispersions having a 1:5 ratio by weight of bicalutamide:polymer were prepared as follows.

0.5g of bicalutamide and 2.5g of polymer were weighed directly into a 250ml round bottom flask and dissolved in 80ml of acetone:dichloromethane (3:1). The solvent was removed on a rotary evaporator or by spray drying. The formulation was placed in a vacuum oven and dried under high vacuum at 40°C for 24 hours.

The formulation was retrieved from the flask and dry milled using a Fritsch mill. The formulation was then dried for a further 24 hours under high vacuum at 40°C.

In order to produce formulations having ratios other than 1:5, weights and volumes in the process should be adjusted so that they are pro-rata to those described above.

#### *In vitro* Dissolution Test

#### (a) Solid Dispersion with Enteric Polymers v. Solid Dispersion With Non-Enteric Polymers

The formulations were weighed into hard gelatin capsules (equivalent to 50mg drug) and dissoluted in 900ml media [either 0.25% sodium dodecyl sulphate solution or pH6.5 buffer] for one hour at 37°C (paddle speed 75rpm). 5ml samples were then removed with a plastic syringe at 5, 10, 20, 30, 45 and 60 minutes. Each sample was centrifuged (14,000rpm) at ambient temperature for 15 minutes and then analysed by HPLC using the following conditions:-

| | |
|---|---|
| Eluent: | 58% ACN/42% water/0.2% formic acid |
| Column: | 15cm Luna 5um, 3mm id column (with guard) |
| Detection wavelength: | 270nm |

(continued)

| | |
|---|---|
| Flow rate: | 1ml/min |
| Temperature: | ambient |
| Injection: | 10ul |
| Retention time: | approximately 2 minutes |

Figures 1 and 2 show the results of *in vitro* dissolution tests performed on the various solid dispersions. As Fig. 1 shows, 100% of bicalutamide in solution was achieved with the HPMCP HP-555, EUDRAGIT L100 and HPMCAS AQOAT LG solid dispersions and supersaturation was maintained over the 60 minute test (ie, no drug precipitation was observed), which is a significant improvement over the conventional tablet. Compare this against the results (Fig. 2) for the PLA:PEG solid dispersion, which did not show any improvement over the conventional tablet formulation. The PEG4000 solid dispersion also was much inferior to the formulations using enteric polymers (Fig. 2), the former achieving only just over 40% of bicalutamide in solution. In addition, reference to Fig. 2 shows that the solid dispersions with METOLOSE 60SH 50cp and HPMC PHARMACOAT 606 only achieved approximately 58% and 70% of bicalutamide in solution.

(b) Solid Dispersions With Varying Ratios of Bicalutamide:HP-55S

Solid dispersions were made with weight ratios of 1:1, 1:2, 1:3, 1:4 and 1:5 bicalutamide:HP-55S. These were tested in the *in vitro* dissolution test, and the results are presented in Fig. 3. A conventional bicalutamide tablet formulation was included for comparison.

As Fig. 3 shows, for all of the formulations comprising HP-55S, 100% of bicalutamide in solution was achieved and supersaturation was maintained over the 60 minute test. These results were superior to the results achieved with the conventional formulation.

***In Vivo* Evaluation**

Oral doses of bicalutamide were administered to fasted dogs (equivalent to 450mg drug)(n=6). The formulations dosed were conventional CASODEX™ tablets and a 1:3 [bicalutamide:HP-55S] solid dispersion. The solid dispersion was prepared as described earlier, however the solvent was removed by spray drying as opposed to rotary evaporation. Each oral dose was followed by 20ml of water. Blood samples were taken pre-dose and post dose at 1, 2, 3, 4, 6, 8, 12, 18, 24, 30, 36, 48, 72, 96, 120, 144, 168 hours. The samples centrifuged at 3000rpm for 15 minutes, the plasma removed into plain blood tubes and stored at -20°C until analysis. Samples were analysed by using a suitable extraction method followed by LC-MS.

Summary of Pharmacokinetic Parameters

| FORMULATION | Cpmax (μg/ml) | Tmax (hours) | AUC (μg/h/ml)* |
|---|---|---|---|
| HP-55S solid dispersion | 13 | 30 | 1504 ± 309 |
| Conventional formulation | 5 | 30 | 500 ± 405 |
| * AUC from 0 to 144 hours | | | |

These data, as well as Fig. 4, show that the bioavailability of bicalutamide is greater with the solid dispersion using the enteric HP-55S polymer. In fact, the AUC measurements show a figure for the HP-55S solid dispersion that is almost 3 times that of the conventional tablet formulation. In addition, $C_{max}$ for the HP-55S solid dispersion is almost 3 times that of the conventional tablet formulation. Furthermore, inter-subject variability in the plasma levels of bicalutamide is lower with the HP-55S solid dispersion than with the conventional tablet formulation (for variability/total AUC, compare a figure of 309/1504 μg/h/ml for theHP-55S solid dispersion against a figure of 405/500 μg/h/ml for the conventional tablet formulation). Formulations according to the present invention display similar improvements over a conventional tablet formulation.

**Examples in accordance with the invention**

**B: Enhancement of therapeutic potential provided by the R-enantiomer**

**(i) At a 1:3 ratio**

A solid dispersion was made that had a 1:3 ratio by weight of R-4'-cyano-α',α',α'-trifluoro-3-(4-fluorophenylsulphonyl)-2-hydroxy-2-methylpropiono-*m*-toluidide (100% of the R-enantiomer): HP-55S enteric polymer. Production was by a spray drying method. A second solid dispersion was also made by a spray drying method, but this solid dispersion had a 1:3 ratio by weight of bicalutamide (ie, racemic 4'-cyano-α',α',α'-trifluoro-3-(4-fluorophenylsulphonyl)-2-hydroxy-2-methylpropiono-*m*-toluidide) : HP-55S.

*In vitro* Dissolution Test

The test was performed following the protocol above. Figure 5 shows a comparison of cumulative % 4'-cyano-α',α',α'-trifluoro-3-(4-fluorophenylsulphonyl)-2-hydroxy-2-methylpropiono-*m*-toluidide released v. time for the two formulations and for a conventional 50mg bicalutamide tablet formulation. As Fig. 5 shows, the solid dispersion according to the invention, which had 100% of the R-enantiomer, displayed enhanced drug release compared to the conventional formulation. The enhancement was similar to that achieved by the bicalutamide solid dispersion.

**(ii) At a 1:1 ratio**

The protocol in part (i) was followed, with the exception that the drug : HP-55S ratio for both formulations was changed to 1:1.

*In vitro* Dissolution Test

The test was performed following the protocol above. Figure 6 shows a comparison of cumulative % 4'-cyano-α',α',α'-trifluoro-3-(4-fluorophenylsulphonyl)-2-hydroxy-2-methylpropiono-*m*-toluidide released v. time for the two formulations with a 1:1 ratio. Again, both solid dispersion formulations displayed enhanced drug release compared to the conventional formulation. Indeed, the formulation according to the invention achieved 100% of drug in solution and supersaturation was maintained over the 60 minute test (ie, no drug precipitation was observed).

**Enhancement of storage stability provided by the R-enantiomer**

Solid dispersion formulations were prepared as in part B(i) above (ie, having a 1:3 ratio of drug: HP-55S).

The storage stability of the formulations was assessed using X-ray diffraction (XRD) as follows. The formulations were placed in sealed glass amber vials and stored at the following conditions, 4°C, 25°C/60%RH, 50°C and 40°C/75%RH (RH, relative humidity) for three months. After three months the samples were removed and analysed by XRD (X-ray diffraction) to determine the presence or absence of crystallinity. The results are presented in the following table.

| Drug:Polymer ratio | Storage condition | Three months XRD |
|---|---|---|
| 1:3 | Initial | X |
| (R/S- drug) | 4°C | X |
| | 25°C/60%RH | X |
| | 50°C | √ |
| | 40°C/75%RH | √ |
| | | |
| 1:3 | Initial | X |
| (R- drug) | 4°C | X |
| | 25°C/60%RH | X |
| | 50°C | X |

(continued)

| Drug:Polymer ratio | Storage condition | Three months XRD |
|---|---|---|
| | 40°C/75%RH | X |
| X = no crystallinity<br>√ = crystallinity | | |

As the results show, no crystallinity was detected after 3 months when the formulation according to the invention was stored under any of the conditions, indicating the superior stability of the formulation. With the bicalutamide (R/S-) formulation, however, the formulation was less stable, as indicated by the presence of crystallinity. The presence of crystallinity in the R/S sample stored at 40°C/75%RH corresponded with a reduction in the dissolution performance of the formulation when tested after 3 months of storage.

**C: Preparation of solid Dispersion for a combination formulation**

**FOR A 1:3 RATIO DRUG TO POLYMER RATIO**

Drug amounts calculated for a dose ratio of 150mg Bicalutamide to 10mg Tamoxifen (10mg tamoxifen = 15.2mg tamoxifen citrate) 0.908g of Bicalutamide and 0.092g of Tamoxifen citrate was weighed directly into a 250ml round bottom flask. 3g of polymer was then added to the flask and dissolved in 120mls of Acetone. The solvent was removed on the rotary evaporator. The formulation was placed in a vacuum oven and dried under high vacuum at 40°C for 24hrs.

The formulation was retrieved from the flask and dry milled using the Fritsch mill (350rpm/15mins). The formulation was then dried for a further 24Hrs under high vacuum at 40°C.

Weights and volumes for other ratios are pro-rata to the above formulation.

**PREPARATION OF A BLEND**

Drug amounts calculated for a dose ratio of 150mg bicalutamide to 10mg tamoxifen (10mg tamoxifen = 15.2mg tamoxifen citrate)

1.8g of a 1:3 bicalutamide/HPMC Phthalate (HP-55S) solid dispersion and 0.04560mg of tamoxifen citrate were blended together in a pestle and mortar.

PH shift test

The formulations were weighed into hard gelatin capsules (equivalent to 50mg bicalutamide). Approx. 1 hour before starting dissolution, 900ml of SGF (Simulated gastric Fluid, pH 1.5) was allowed to equilibrate to 37°C (paddle speed 75rpm). The capsules were added to the media and after 1 hour a zero time point was taken. The sample was centrifuged (14,000rpm) at ambient temperature for 15 minutes and then analysed by HPLC. Immediately, 18mls of $KH_2PO_4$ 2.5M/16.72% NaOH was added to each pot and the timer started again to record the subsequent 1 hour dissolution at pH 6.5. 5ml samples were then removed with a plastic syringe at 5, 15, 30, 45 and 60 mins. Each sample was centrifuged (14,000rpm) at ambient temperature for 15 minutes and then analysed by HPLC using the following conditions: -

| | |
|---|---|
| Eluen | 58% ACN/42% water/0.2% formic acid |
| Column: | 15cm Luna 5um, 3mm id column (with guard) |
| Detection wavelength: | 270nm |
| Flow rate: | 1ml/min |
| Temperature: | ambient |
| Injection: | 10ul |
| Retention time: | Tamoxifen approximately 1 minute<br>Bicalutamide approximately 2 minutes |

Figures 7 and 8 show the results of *in vitro* dissolution test performed on the bicalutamide/tamoxifen solid dispersion and the 1:3 bicalutamide/HP-55S solid dispersion and tamoxifen blend. Figure 7 shows the cumulative % released of bicalutamide and Figure 8 the cumulative % released of tamoxifen. Figure 7 shows that post shift >90% of bicalutamide in solution was achieved with both formulations and supersaturation was maintained over the 60 minute

test at pH 6.5 (ie, no drug precipitation was observed), which is a significant improvement over the conventional tablet. Figure 8 shows post shift approx. 80% of tamoxifen in solution with both formulations, this is equivalent to the amount seen in solution for tamoxifen citrate alone under these test conditions.

**D: Crystallinity assessment of formulations**

The following samples were prepared and analysed by X-ray powder diffraction (XRPD):

■ A- Solid dispersion with drug:HP-55S ratio of 1:3. Dispersion prepared from a solution of bicalutamide and tamoxifen citrate. Final composition: bicalutamide 22.6%, tamoxifen citrate 2.4%, HP-55S 75% in final dispersion.

■ B - Physical Blend of tamoxifen citrate and a solid dispersion containing 1:3 bicalutamide/HP-55S. Final composition: bicalutamide 24.4%, tamoxifen citrate 2.4%, HP-55S 73.2%.

■ C- tamoxifen citrate (purchased from Heumann)

X-ray diffraction analysis was performed according to standard methods which can be found in e.g. Bunn, C. W. (1948), Chemical Crystallography, Clarendon Press, London; or Klug, H.P. & Alexander, L. E. (1974), X-Ray Diffraction Procedures, John Wiley and Sons, New York.

Results

XRPD patterns are given in Figure 9. It can be seen that bicalutamide was amorphous in both formulations (samples A and B) while tamoxifen citrate (sample C) was crystalline. Close examination of the XRPD patterns of samples A and B reveals two small peaks in the XRPD of sample B, which are not present in the XRPD of sample A. These peaks correspond to the most intense tamoxifen citrate peaks and their presence indicates that crystalline tamoxifen citrate is detected in sample B, but not detected in sample A.
It can be concluded that the bicalutamide in both preparations is amorphous. The limit of detection of tamoxifen citrate in these dispersions has not been established; however we can say that that the physical mixture of tamoxifen citrate with a bicalutamide/HP-55S dispersion still contains some crystalline tamoxifen citrate, while a dispersion of both drugs in HP-55S has no detectable crystalline tamoxifen citrate.

## Claims

1. A pharmaceutical product comprising 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in a solid dispersion comprising an enteric polymer having a $pK_a$ from 3 to 6, the product further comprising an anti-oestrogen and/or an aromatase inhibitor.

2. The pharmaceutical product according to claim 1, comprising 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in a solid dispersion comprising an enteric polymer having a $pK_a$ from 3 to 6, the product further comprising an anti-oestrogen.

3. The pharmaceutical product according to claim 1 or 2 wherein the anti-oestrogen is in solid dispersion with the enteric polymer.

4. The pharmaceutical product according to claim 1, 2 or 3, wherein the anti-oestrogen is tamoxifen or a pharmaceutically acceptable salt or solvate thereof.

5. The pharmaceutical product according to any preceding claim, wherein the 4'-cyano-α',α',α'-trifluoro-3-(4-fluorophenylsulphonyl)-2-hydroxy-2-methylpropiono-*m*-toluidide and anti-oestrogen are provided in a ratio of 25 to 350 : 0.5 to 100 respectively.

6. The pharmaceutical product according to claim 1, comprising 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in a solid dispersion comprising an enteric polymer having a $pK_a$ from 3 to 6, the product further comprising an aromatase inhibitor.

7. The pharmaceutical product according to claim 1 or 6, wherein the aromatase inhibitor is in solid dispersion with the enteric polymer.

8. The pharmaceutical product of claim 1, 6 or 7, wherein the aromatase inhibitor is selected from anastrazole, letrozole and exemestane or a pharmaceutically acceptable salt or solvate thereof.

9. The pharmaceutical product according to any one of claims 1, 6, 7 or 8, wherein the 4'-cyano-α',α',α'-trifluoro-3-(4-fluorophenylsulphonyl)-2-hydroxy-2-methylpropiono-*m*-toluidide and aromatase inhibitor are provided in a ratio of 25 to 350 : 0.005 to 100 respectively.

10. The pharmaceutical product according to any preceding claim, wherein >50% of the 4'-cyano-α',α',α'-trifluoro-3-(4-fluorophenylsulphonyl)-2-hydroxy-2-methylpropiono-*m*-toluidide is provided in the form of the R-enantiomer.

11. The pharmaceutical product according to claim 9, wherein about >50%, ≥60%, ≥70%, ≥80%, ≥85%, ≥90%, ≥95%, ≥98% or ≥99%, or substantially 100% of the 4'-cyano-α',α',α'-trifluoro-3-(4-fluorophenylsulphonyl)-2-hydroxy-2-methylpropiono-*m*-toluidide is provided in the form of the R-enantiomer.

12. The pharmaceutical product according to any preceding claim, wherein the enteric polymer is selected from the group consisting of: hydroxypropyl methylcellulose acetate succinate (HPMCAS), hydroxpropyl methylcellulose acetate pthalate, hydroxypropyl methylcellulose acetate, hydroxypropyl methylcellulose succinate a methacrylic acid copolymer, polyvinyl acetate phthalate (PVAP), cellulose acetate phthalate (CAP), methylcellulose acetate phthalate, ethyl cellulose acetate phthalate, hydroxypropyl cellulose acetate phthalate, hydroxypropyl methylcellulose phthalate (HPMCP), cellulose proprionate pthalate, hydroxypropyl cellulose butyrate pthalate, hydroxypropyl cellulose acetate pthalate succinate, hydroxypropyl methylcellulose trimellitate, cellulose acetate trimellitate (CAT), methylcellulose acetate trimellitate, ethyl cellulose acetate trimellitate, hydroxypropyl cellulose acetate trimellitate, hydroxypropyl methylcellulose acetate trimellitate, hydroxypropyl cellulose acetate trimellitate succinate, cellulose proprionate trimellitate, cellulose butyrate trimellitate, cellulose acetate terepthalate and cellulose acetate isopthalate, or any combination thereof.

13. The pharmaceutical product according to claim 12, wherein the enteric polymer is selected from the group consisting of: HPMCP grade HP-50, HPMCP grade HP-55, HPMCP grade HP-55S, HPMCAS grade AS-LF, HPMCAS grade AS-MF, HPMCAS grade AS-HF, HPMCAS grade AS-LG, HPMCAS grade AS-MG, HPMCAS grade AS-HG, methacrylic acid copolymer grade A and methacrylic acid copolymer grade B.

14. The pharmaceutical product according to claim 13, wherein the enteric polymer is selected from the group consisting of: HPMCP grade HP-55S, HPMCAS grade AS-LG and methacrylic acid copolymer grade A.

15. The pharmaceutical product according to any preceding claim, wherein the weight ratio of 4'-cyano-α',α',α'-trifluoro-3-(4-fluorophenylsulphonyl)-2-hydroxy-2-methylpropiono-*m*-toluidide : enteric polymer is from 1:0.25 to 1:10.

16. The pharmaceutical product according to any preceding claim, wherein the solid dispersion comprises a wetting agent.

17. The pharmaceutical product according to claim 1 or 2, in a dosage form comprising 25 to 1000 mg of 4'-cyano-α', α',α'-trifluoro-3-(4-fluorophenylsulphonyl)-2-hydroxy-2-methylpropiono-*m*-toluidide, the dose further comprising an anti-oestrogen.

18. The product according to claim 17, wherein the anti-oestrogen is as defined in claim 3 or 4.

19. The product according to claim 17 or 18, wherein the anti-oestrogen is provided in an amount of 0.5 to 200 mg.

20. The pharmaceutical product according to claim 1 or 6 in a dosage form comprising 25 to 1000 mg of 4'-cyano-α', α',α'-trifluoro-3-(4-fluorophenylsulphonyl)-2-hydroxy-2-methylpropiono-*m*-toluidide, the dose further comprising an aromatase inhibitor.

21. The product according to claim 20, wherein the aromatase inhibitor is as defined in claim 7 or 8.

22. The product according to claim 20 or 21, wherein the aromatase inhibitor is provided in an amount of 0.005 to 200 mg.

**23.** The product according to any one of claims 17 to 22, wherein >50% of the 4'-cyano-α',α',α'-trifluoro-3-(4-fluorophenylsulphonyl)-2-hydroxy-2-methylpropiono-m-toluidide is provided in the form of the R-enantiomer.

**24.** The product according to claim 23, wherein about >50%, ≥60%, ≥70%, ≥80%, ≥85%, ≥90%, ≥95%, ≥98% or ≥99%, or substantially 100% of the 4'-cyano-α',α',α'-trifluoro-3-(4-fluorophenylsulphonyl)-2-hydroxy-2-methylpropiono-*m*-toluidide is provided in the form of the R-enantiomer.

**25.** The product according to any one of claims 17 to 24, wherein the enteric polymer is as defined in any one of claims 11 to 13.

**26.** The product according to any one of claims 17 to 25, wherein the weight ratio of 4'-cyano-α',α',α'-trifluoro-3-(4-fluorophenylsulphonyl)-2-hydroxy-2-methylpropiono-*m*-toluidide : enteric polymer is from 1:0.25 to 1:10.

**27.** The product according to any one of claims 17 to 26, wherein the solid dispersion comprises a wetting agent.

**28.** Use in the manufacture of a pharmaceutical product of an anti-oestrogen and 4'-cyano-α',α',α'-trifluoro-3-(4-fluorophenylsulphonyl)-2-hydroxy-2-methylpropiono-*m*-toluidide or a pharmaceutically acceptable salt or solvate thereof, for simultaneous or sequential administration to a patient, for increasing the bioavailability of 4'-cyano-α',α',α'-trifluoro-3-(4-fluorophenylsulphonyl)-2-hydroxy-2-methylpropiono-*m*-toluidide in the patient and treating and/or preventing at least one side effect selected from gynaecomastia, breast tenderness, hot flushes, impotence and reduction in libido, the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6.

**29.** Use in the manufacture of a pharmaceutical product of an anti-oestrogen and 4'-cyano-α',α',α'-trifluoro-3-(4-fluorophenylsulphonyl)-2-hydroxy-2-methylpropiono-m-toluidide or a pharmaceutically acceptable salt or solvate thereof, for simultaneous or sequential administration to patients, for reducing inter-patient variability in plasma concentrations of 4'-cyano-α',α',α'-trifluoro-3-(4-fluorophenylsulphonyl)-2-hydroxy-2-methylpropiono-*m*-toluidide in the patient and treating and/or preventing at least one side effect selected from gynaecomastia, breast tenderness, hot flushes, impotence and reduction in libido, the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6.

**30.** Use in the manufacture of a pharmaceutical product of an anti-oestrogen and 4'-cyano-α',α',α'-trifluoro-3-(4-fluorophenylsulphonyl)-2-hydroxy-2-methylpropiono-m-toluidide or a pharmaceutically acceptable salt or solvate thereof, for simultaneous or sequential administration to a patient, for treating and/or reducing the risk of prostate cancer in the patient and treating and/or preventing at least one side effect selected from gynaecomastia, breast tenderness, hot flushes, impotence and reduction in libido, the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6.

**31.** The use according to claim 28, 29 or 30, wherein the anti-oestrogen is tamoxifen or a pharmaceutically acceptable salt or solvate thereof.

**32.** The use according to claim 28, 29, 30 or 31, wherein the 4'-cyano-α',α',α'-trifluoro-3-(4-fluorophenylsulphonyl)-2-hydroxy-2-methylpropiono-m-toluidide and anti-oestrogen are provided in a ratio of 25 to 350 : 0.5 to 100 respectively.

**33.** Use in the manufacture of a pharmaceutical product of an aromatase inhibitor and 4'-cyano-α',α',α'-trifluoro-3-(4-fluorophenylsulphonyl)-2-hydroxy-2-methylpropiono-*m*-toluidide or a pharmaceutically acceptable salt or solvate thereof, for simultaneous or sequential administration to a patient, for increasing the bioavailability of 4'-cyano-α',α',α'-trifluoro-3-(4-fluorophenylsulphonyl)-2-hydroxy-2-methylpropiono-*m*-toluidide in the patient and treating and/or preventing at least one side effect selected from gynaecomastia, breast tenderness, hot flushes, impotence and reduction in libido, the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6.

**34.** Use in the manufacture of a pharmaceutical product of an aromatase inhibitor and 4'-cyano-α',α',α'-trifluoro-3-(4-fluorophenylsulphonyl)-2-hydroxy-2-methylpropiono-m-toluidide or a pharmaceutically acceptable salt or solvate thereof, for simultaneous or sequential administration to patients, for reducing inter-patient variability in plasma concentrations of 4'-cyano-α',α',α'-trifluoro-3-(4-fluorophenylsulphonyl)-2-hydroxy-2-methylpropiono-*m*-toluidide in the patient and treating and/or preventing at least one side effect selected from gynaecomastia, breast tenderness, hot flushes, impotence and reduction in libido, the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6.

**35.** Use in the manufacture of a pharmaceutical product of an aromatase inhibitor and 4'-cyano-α',α',α'-trifluoro-3-(4-fluorophenylsulphonyl)-2-hydroxy-2-methylpropiono-*m*-toluidide or a pharmaceutically acceptable salt or solvate thereof, for simultaneous or sequential administration to a patient, for treating and/or reducing the risk of prostate cancer in the patient and treating and/or preventing at least one side effect selected from gynaecomastia, breast tenderness, hot flushes, impotence and reduction in libido, the 4'-cyano-α',α',α'-trifluoro-3-(4-fluorophenylsulphonyl)-2-hydroxy-2-methylpropiono-*m*-toluidide being in solid dispersion with an enteric polymer having a $pK_a$ from 3 to 6.

**36.** The use according to any one of claims 33 to 35, wherein the aromatase inhibitor is selected from anastrazole, letrozole and exemestane or a pharmaceutically acceptable salt or solvate thereof.

**37.** The use according to any one of claims 33 to 36, wherein the 4'-cyano-α',α',α'-trifluoro-3-(4-fluorophenylsulphonyl)-2-hydroxy-2-methylpropiono-*m*-toluidide and aromatase inhibitor are provided in a ratio of 25 to 350 : 0.005 to 100 respectively.

**38.** The use according to any of claims 28 to 37, wherein about >50%, ≥60%, ≥70%, ≥80%, ≥85%, ≥90%, ≥95%, ≥98% or ≥99%, or substantially 100% of the 4'-cyano-α',α',α'-trifluoro-3-(4-fluorophenylsulphonyl)-2-hydroxy-2-methylpropiono-*m*-toluidide is provided in the form of the R-enantiomer.

**39.** The pharmaceutical product according to claim 1, comprising 4'-cyano-α',α',α'-trifluoro-3-(4-fluorophenylsulphonyl)-2-hydroxy-2-methylpropiono-*m*-toluidide, or a pharmaceutically acceptable salt or solvate thereof, in a solid dispersion comprising HPMCP grade HP-55S, the product further comprising tamoxifen.

**40.** A pharmaceutical product according to claim 39, wherein about >50%, ≥60%, ≥70%, ≥80%, ≥85%, ≥90%, ≥95%, ≥98% or ≥99%, or substantially 100% of the 4'-cyano-α',α',α'-trifluoro-3-(4-fluorophenylsulphonyl)-2-hydroxy-2-methylpropiono-*m*-toluidide is provided in the form of the R-enantiomer.

**41.** The product according to any of claim 1 to 27, 39 and 40, wherein, at least 30%, 40%, 50%, 75%, 90%, 95% or 99% of the 4'-cyano-α',α',α'-trifluoro-3-(4-fluorophenylsulphonyl)-2-hydroxy-2-methylpropiono-*m*-toluidide is in amorphous form.

**42.** The use according to any of claims 28 to 38, wherein at least 30%, 40%, 50%, 75%, 90%, 95% or 99% of the 4'-cyano-α',α',α'-trifluoro-3-(4-fluorophenylsulphonyl)-2-hydroxy-2-methylpropiono-*m*-toluidide is in amorphous form.

**Patentansprüche**

**1.** Pharmazeutisches Produkt, umfassend 4'-Cyano-α',α',α'-trifluor-3-(4-fluorphenylsulfonyl)-2-hydroxy-2-methylpropiono-m-toluidid oder ein pharmazeutisch annehmbares Salz oder Solvat davon in einer festen Dispersion, die ein magensaftresistentes Polymer mit einem $pK_a$ von 3 bis 6 enthält, wobei das Produkt ferner ein Anti-Östrogen und/oder einen Aromataseinhibitor enthält.

**2.** Pharmazeutisches Produkt nach Anspruch 1, umfassend 4'-Cyano-α',α',α'-trifluor-3-(4-fluorphenylsulfonyl)-2-hydroxy-2-methylpropiono-*m*-toluidid oder ein pharmazeutisch annehmbares Salz oder Solvat davon in einer festen Dispersion, die ein magensaftresistentes Polymer mit einem $pK_a$ von 3 bis 6 enthält, wobei das Produkt ferner ein Anti-Östrogen enthält.

**3.** Pharmazeutisches Produkt nach Anspruch 1 oder 2, wobei das Anti-Östrogen sich in einer festen Dispersion mit dem magensaftresistenten Polymer befindet.

**4.** Pharmazeutisches Produkt nach Anspruch 1, 2 oder 3, wobei das Anti-Östrogen Tamoxifen oder ein pharmazeutisch annehmbares Salz oder Solvat davon ist.

**5.** Pharmazeutisches Produkt nach einem vorhergehenden Anspruch, wobei 4'-Cyano-α',α',α'-trifluor-3-(4-fluorphenylsulfonyl)-2-hydroxy-2-methylpropiono-*m*-toluidid und das Anti-Östrogen in einem Verhältnis von 25 bis 350:0,5 bis 100 bereitgestellt werden.

**6.** Pharmazeutisches Produkt nach Anspruch 1, umfassend 4'-Cyano-α',α',α'-trifluor-3-(4-fluorphenylsulfonyl)-2-hydroxy-2-methylpropiono-*m*-toluidid oder ein pharmazeutisch annehmbares Salz oder Solvat davon in einer festen Dispersion, die ein magensaftresistentes Polymer mit einem $pK_a$ von 3 bis 6 enthält, wobei das Produkt ferner einen Aromataseinhibitor enthält.

**7.** Pharmazeutisches Produkt nach Anspruch 1 oder 6, wobei der Aromataseinhibitor sich in einer festen Dispersion mit dem magensaftresistenten Polymer befindet.

**8.** Pharmazeutisches Produkt nach Anspruch 1, 6 oder 7, wobei der Aromataseinhibitor aus Anastrazol, Letrozol und Exemestan oder einem pharmazeutisch annehmbaren Salz oder Solvat davon ausgewählt ist.

**9.** Pharmazeutisches Produkt nach einem der Ansprüche 1, 6, 7 oder 8, wobei 4'-Cyano-α',α',α'-trifluor-3-(4-fluorphenylsulfonyl)-2-hydroxy-2-methylpropiono-m-toluidid und der Aromataseinhibitor in einem Verhältnis von 25 bis 350: 0,005 bis 100 bereitgestellt werden.

**10.** Pharmazeutisches Produkt nach einem vorhergehenden Anspruch, wobei > 50% des 4'-Cyano-α',α',α'-trifluor-3-(4-fluorphenylsulfonyl)-2-hydroxy-2-methylpropiono-m-toluidids in Form des R-Enantiomers bereitgestellt werden.

**11.** Pharmazeutisches Produkt nach Anspruch 9, wobei etwa > 50%, ≥ 60%, ≥ 70%, ≥ 80%, ≥ 85%, ≥ 90%, ≥ 95%, ≥ 98°s oder ≥ 99% oder im Wesentlichen 100% des 4'-Cyano-α',α',α'-trifluor-3-(4-fluorphenylsulfonyl)-2-hydroxy-2-methylpropiono-*m*-toluidids in Form des R-Enantiomers bereitgestellt wird.

**12.** Pharmazeutisches Produkt nach einem vorhergehenden Anspruch, wobei das magensaftresistente Polymer aus der folgenden Gruppe ausgewählt ist: Hydroxypropylmethylcelluloseacetatsuccinat (HPMCAS), Hydroxypropylmethylcelluloseacetatphthalat, Hydroxypropylmethylcelluloseacetat, Hydroxypropylmethylcellulosesuccinat, einem Methacrylsäure-Copolymer, Polyvinylacetatphthalat (PVAP), Celluloseacetatphthalat (CAP), Methylcelluloseacetatphthalat, Ethylcelluloseacetatphthalat, Hydroxypropylcelluloseacetatphthalat, Hydroxypropylmethylcellulosephthalat (HPMCP), Cellulosepropionatphthalat, Hydroxypropylcellulosebutyratphthalat, Hydroxypropylcelluloseacetatphthalatsuccinat, Hydroxypropylmethylcellulosetrimellitat, Celluloseacetattrimellitat (CAT), Methylcelluloseacetattrimellitat, Ethylcelluloseacetattrimellitat, Hydroxypropylcelluloseacetattrimellitat, Hydroxypropylmethylcelluloseacetattrimellitat, Hydroxypropylcelluloseacetattrimellitatsuccinat, Cellulosepropionattrimellitat, Cellulosebutyrattrimellitat, Celluloseacetatterephthalat und Celluloseacetatisophthalat oder jeder Kombination davon.

**13.** Pharmazeutisches Produkt nach Anspruch 12, wobei das magensaftresistente Polymer aus der folgenden Gruppe ausgewählt ist: HPMCP Qualität HP-50, HPMCP Qualität HP-55, HPMCP Qualität HP-55S, HPMCAS Qualität AS-LF, HPMCAS Qualität AS-MF, HPMCAS Qualität AS-HF, HPMCAS Qualität AS-LG, HPMCAS Qualität AS-MG, HPMCAS Qualität AS-HG, Methacrylsäure-Copolymer Qualität A und Methacrylsäure-Copolymer Qualität B.

**14.** Pharmazeutisches Produkt nach Anspruch 13, wobei das magensaftresistente Polymer aus der folgenden Gruppe ausgewählt ist: HPMCP Qualität HP-55S, HPMCAS Qualität AS-LG und Methacrylsäure-Copolymer Qualität A.

**15.** Pharmazeutisches Produkt nach einem vorhergehenden Anspruch, wobei das Gewichtsverhältnis von 4'-Cyano-α',α',α'-trifluor-3-(4-fluorphenylsulfonyl)-2-hydroxy-2-methylpropiono-*m*-toluidid:magensaftresistentem Polymer von 1:0,25 bis 1:10 reicht.

**16.** Pharmazeutisches Produkt nach einem vorhergehenden Anspruch, wobei die feste Dispersion ein Benetzungsmittel enthält.

**17.** Pharmazeutisches Produkt nach Anspruch 1 oder 2 in einer Dosierungsform, die 25 bis 1000 mg 4'-Cyano-α',α',α'-trifluor-3-(4-fluorphenylsulfonyl)-2-hydroxy-2-methylpropiono-m-toluidid enthält, wobei die Dosis ferner ein Anti-Östrogen enthält.

**18.** Produkt nach Anspruch 17, wobei das Anti-Östrogen wie in Anspruch 3 oder 4 definiert ist.

**19.** Produkt nach Anspruch 17 oder 18, wobei das Anti-Östrogen in einer Menge von 0,5 bis 200 mg bereitgestellt wird.

**20.** Pharmazeutisches Produkt nach Anspruch 1 oder 6 in einer Dosierungsform, die 25 bis 1000 mg 4'-Cyano-α',α',α'-trifluor-3-(4-fluorphenylsulfonyl)-2-hydroxy-2-methylpropiono-m-toluidid enthält, wobei die Dosis ferner einen Aromataseinhibitor enthält.

21. Produkt nach Anspruch 20, wobei der Aromataseinhibitor wie in Anspruch 7 oder 8 definiert ist.

22. Produkt nach Anspruch 20 oder 21, wobei der Aromataseinhibitor in einer Menge von 0,005 bis 200 mg bereitgestellt wird.

23. Produkt nach einem der Ansprüche 17 bis 22, wobei > 50% des 4'-Cyano-α',α',α'-trifluor-3-(4-fluorphenylsulfonyl)-2-hydroxy-2-methylpropiono-*m*-toluidids in Form des R-Enantiomers bereitgestellt werden.

24. Produkt nach Anspruch 23, wobei etwa > 50%, ≥ 60%, ≥ 70%, ≥ 80%, ≥ 85%, ≥ 90%, ≥ 95%, ≥ 98% oder ≥ 99% oder im Wesentlichen 100% des 4'-Cyano-α',α',α'-trifluor-3-(4-fluorphenylsulfonyl)-2-hydroxy-2-methylpropiono-*m*-toluidids in Form des R-Enantiomers bereitgestellt werden.

25. Produkt nach einem der Ansprüche 17 bis 24, wobei das magensaftresistente Polymer wie in einem der Ansprüche 11 bis 13 definiert ist.

26. Produkt nach einem der Ansprüche 17 bis 25, wobei das Gewichtsverhältnis von 4'-Cyano-α',α',α'-trifluor-3-(4-fluorphenylsulfonyl)-2-hydroxy-2-methylpropiono-m-toluidid:magensaftresistentem Polymer von 1:0,25 bis 1:10 reicht.

27. Produkt nach einem der Ansprüche 17 bis 26, wobei die feste Dispersion ein Benetzungsmittel enthält.

28. Verwendung eines Anti-Östrogens und von 4'-Cyano-α',α',α'-trifluor-3-(4-fluorphenylsulfonyl)-2-hydroxy-2-methylpropiono-m-toluidid oder eines pharmazeutisch annehmbaren Salzes oder Solvats davon zur Herstellung eines pharmazeutischen Produkts zur gleichzeitigen oder aufeinander folgenden Verabreichung an einen Patienten zur Erhöhung der Bioverfügbarkeit von 4'-Cyano-α',α',α'-trifluor-3-(4-fluorphenylsulfonyl)-2-hydroxy-2-methylpropiono-m-toluidid in dem Patienten und zur Behandlung und/oder Vorbeugung mindestens einer Nebenwirkung, die aus Gynäkomastie, Brustberührungsschmerz, Hitzewellen, Impotenz und Verringerung der Libido ausgewählt ist, wobei 4'-Cyano-α',α',α'-trifluor-3-(4-fluorphenylsulfonyl)-2-hydroxy-2-methylpropiono-*m*-toluidid in einer festen Dispersion mit einem magensaftresistenten Polymer mit einem $pK_a$ von 3 bis 6 vorliegt.

29. Verwendung eines Anti-Östrogens und von 4'-Cyano-α',α',α'-trifluor-3-(4-fluorphenylsulfonyl)-2-hydroxy-2-methylpropiono-m-toluidid oder eines pharmazeutisch annehmbaren Salzes oder Solvats davon zur Herstellung eines pharmazeutischen Produkts zur gleichzeitigen oder aufeinander folgenden Verabreichung an Patienten zur Verringerung der Inter-Patienten-Schwankung in den Plasmakonzentrationen von 4'-Cyano-α',α',α'-trifluor-3-(4-fluorphenylsulfonyl)-2-hydroxy-2-methylpropiono-m-toluidid in dem Patienten und zur Behandlung und/oder Vorbeugung mindestens einer Nebenwirkung, die aus Gynäkomastie, Brustberührungsschmerz, Hitzewellen, Impotenz und Verringerung der Libido ausgewählt ist, wobei 4'-Cyano-α',α',α'-trifluor-3-(4-fluorphenylsulfonyl)-2-hydroxy-2-methylpropiono-m-toluidid in einer festen Dispersion mit einem magensaftresistenten Polymer mit einem $pK_a$ von 3 bis 6 vorliegt.

30. Verwendung eines Anti-Östrogens und von 4'-Cyano-α',α',α'-trifluor-3-(4-fluorphenylsulfonyl)-2-hydroxy-2-methylpropiono-m-toluidid oder eines pharmazeutisch annehmbaren Salzes oder Solvats davon zur Herstellung eines pharmazeutischen Produkts zur gleichzeitigen oder aufeinander folgenden Verabreichung an einen Patienten zur Behandlung und/oder Verringerung des Risikos von Prostatakrebs bei dem Patienten und zur Behandlung und/oder Vorbeugung mindestens einer Nebenwirkung, die aus Gynäkomastie, Brustberührungsschmerz, Hitzewellen, Impotenz und Verringerung der Libido ausgewählt ist, wobei 4'-Cyano-α',α',α'-trifluor-3-(4-fluorphenylsulfonyl)-2-hydroxy-2-methylpropiono-m-toluidid in einer festen Dispersion mit einem magensaftresistenten Polymer mit einem $pK_a$ von 3 bis 6 vorliegt.

31. Verwendung nach Anspruch 28, 29 oder 30, wobei das Anti-Östrogen Tamoxifen oder ein pharmazeutisch annehmbares Salz oder Solvat davon ist.

32. Verwendung nach Anspruch 28, 29, 30 oder 31, wobei 4'-Cyano-α',α',α'-trifluor-3-(4-fluorphenylsulfonyl)-2-hydroxy-2-methylpropiono-*m*-toluidid und Anti-Östrogen in einem Verhältnis von 25 bis 350:0,5 bis 100 bereitgestellt werden.

33. Verwendung eines Aromataseinhibitors und von 4'-Cyano-α',α',α'-trifluor-3-(4-fluorphenylsulfonyl)-2-hydroxy-2-methylpropiono-m-toluidid oder eines pharmazeutisch annehmbaren Salzes oder Solvats davon zur Herstellung eines pharmazeutischen Produkts zur gleichzeitigen oder aufeinander folgenden Verabreichung an einen Patienten zur Erhöhung der Bioverfügbarkeit von 4'-Cyano-α',α',α'-trifluor-3-(4-fluorphenylsulfonyl)-2-hydroxy-2-methylpropiono-m-toluidid in dem Patienten und zur Behandlung und/oder Vorbeugung mindestens einer Nebenwirkung, die aus Gynäkomastie, Brustberührungsschmerz, Hitzewellen, Impotenz und Verringerung der Libido ausgewählt ist, wobei 4'-Cyano-α',α',α'-trifluor-3-(4-fluorphenylsulfonyl)-2-hydroxy-2-methylpropiono-m-toluidid in einer festen Dispersion mit einem magensaftresistenten Polymer mit einem $pK_a$ von 3 bis 6 vorliegt.

**34.** Verwendung eines Aromataseinhibitors und von 4'-Cyano-α',α',α'-trifluor-3-(4-fluorphenylsulfonyl)-2-hydroxy-2-methylpropiono-*m*-toluidid oder eines pharmazeutisch annehmbaren Salzes oder Solvats davon zur Herstellung eines pharmazeutischen Produkts zur gleichzeitigen oder aufeinander folgenden Verabreichung an Patienten zur Verringerung der Inter-Patienten-Schwankung in den Plasmakonzentrationen von 4'-Cyano-α',α',α'-trifluor-3-(4-fluorphenylsulfonyl)-2-hydroxy-2-methylpropiono-m-toluidid in dem Patienten und zur Behandlung und/oder Vorbeugung mindestens einer Nebenwirkung, die aus Gynäkomastie, Brustberührungsschmerz, Hitzewellen, Impotenz und Verringerung der Libido ausgewählt ist, wobei 4'-Cyano-α',α',α'-trifluor-3-(4-fluorphenylsulfonyl)-2-hydroxy-2-methylpropiono-m-toluidid in einer festen Dispersion mit einem magensaftresistenten Polymer mit einem $pK_a$ von 3 bis 6 vorliegt.

**35.** Verwendung eines Aromataseinhibitors und von 4'-Cyano-α',α',α'-trifluor-3-(4-fluorphenylsulfonyl)-2-hydroxy-2-methylpropiono-*m*-toluidid oder eines pharmazeutisch annehmbaren Salzes oder Solvats davon zur Herstellung eines pharmazeutischen Produkts zur gleichzeitigen oder aufeinander folgenden Verabreichung an einen Patienten zur Behandlung und/oder Verringerung des Risikos von Prostatakrebs bei dem Patienten und zur Behandlung und/oder Vorbeugung mindestens einer Nebenwirkung, die aus Gynäkomastie, Brustberührungsschmerz, Hitzewellen, Impotenz und Verringerung der Libido ausgewählt ist, wobei 4'-Cyano-α',α',α'-trifluor-3-(4-fluorphenylsulfonyl)-2-hydroxy-2-methylpropiono-m-toluidid in einer festen Dispersion mit einem magensaftresistenten Polymer mit einem $pK_a$ von 3 bis 6 vorliegt.

**36.** Verwendung nach einem der Ansprüche 33 bis 35, wobei der Aromataseinhibitor aus Anastrazol, Letrozol und Exemestan oder einem pharmazeutisch annehmbaren Salz oder Solvat davon ausgewählt ist.

**37.** Verwendung nach einem der Ansprüche 33 bis 36, wobei 4'-Cyano-α',α',α'-trifluor-3-(4-fluorphenylsulfonyl)-2-hydroxy-2-methylpropiono-*m*-toluidid und der Aromataseinhibitor in einem Verhältnis von 25 bis 350:0,005 bis 100 bereitgestellt werden.

**38.** Verwendung nach einem der Ansprüche 28 bis 37, wobei etwa > 50%, ≥ 60%, ≥ 70%, ≥ 80%, ≥ 85%, ≥ 90%, ≥ 95%, ≥ 98% oder ≥ 99% oder im Wesentlichen 100% des 4'-Cyano-α',α',α'-trifluor-3-(4-fluorphenylsulfonyl)-2-hydroxy-2-methylpropiono-*m*-toluidids in Form des R-Enantiomers bereitgestellt werden.

**39.** Pharmazeutisches Produkt nach Anspruch 1, umfassend 4'-Cyano-α',α',α'-trifluor-3-(4-fluorphenylsulfonyl)-2-hydroxy-2-methylpropiono-*m*-toluidid oder ein pharmazeutisch annehmbares Salz oder Solvat davon in einer festen Dispersion, die HPMCP Qualität HP-55S enthält, wobei das Produkt ferner Tamoxifen enthält.

**40.** Pharmazeutisches Produkt nach Anspruch 39, wobei etwa > 50%, ≥ 60%, ≥ 70%, ≥ 80%, ≥ 85%, ≥ 90%, ≥ 95%, ≥ 98% oder ≥ 99% oder im Wesentlichen 100% des 4'-Cyano-α',α',α'-trifluor-3-(4-fluorphenylsulfonyl)-2-hydroxy-2-methylpropiono-*m*-toluidids in Form des R-Enantiomers bereitgestellt werden.

**41.** Produkt nach einem der Ansprüche 1 bis 27, 39 und 40, wobei mindestens 30%, 40%, 50%, 75%, 90%, 95% oder 99% des 4'-Cyano-α',α',α'-trifluor-3-(4-fluorphenylsulfonyl)-2-hydroxy-2-methylpropiono-*m*-toluidids in amorpher Form vorliegen.

**42.** Verwendung nach einem der Ansprüche 28 bis 38, wobei mindestens 30%, 40%, 50%, 75%, 90%, 95% oder 99% des 4'-Cyano-α',α',α'-trifluor-3-(4-fluorphenylsulfonyl)-2-hydroxy-2-methylpropiono-m-toluidids in amorpher Form vorliegen.

## Revendications

**1.** Produit pharmaceutique comprenant du 4'-cyano-α',α',α'-trifluoro-3-(4-fluorophénylsulfonyl)-2-hydroxy-2-méthylpropiono-m-toluidide, ou un sel ou un solvate pharmaceutiquement acceptable de celui-ci, dans une dispersion solide comprenant un polymère entérique avec un $pK_a$ allant de 3 à 6, le produit comprenant en outre un antioestrogène et/ou un inhibiteur de l'aromatase.

**2.** Produit pharmaceutique selon la revendication 1, comprenant du 4'-cyano-α',α',α'-trifluoro-3-(4-fluorophénylsulfonyl)-2-hydroxy-2-méthylpropiono-m-toluidide, ou un sel ou un solvate pharmaceutiquement acceptable de celui-ci, dans une dispersion solide comprenant un polymère entérique avec un $pK_a$ allant de 3 à 6, le produit comprenant en outre un antioestrogène.

**3.** Produit pharmaceutique selon l'une des revendications 1 ou 2, dans lequel l'antioestrogène est dans une dispersion solide avec le polymère entérique.

**4.** Produit pharmaceutique selon l'une des revendications 1, 2 ou 3, dans lequel l'antioestrogène est du tamoxifène, ou un sel ou un solvate pharmaceutiquement acceptable de celui-ci.

**5.** Produit pharmaceutique selon l'une quelconque des revendications précédentes, dans lequel le 4'-cyano-α',α',α'-trifluoro-3-(4-fluorophénylsulfonyl)-2-hydroxy-2-méthylpropiono-m-toluidide et l'antioestrogène sont présents dans un rapport de 25 à 350 pour 0,5 à 100, respectivement.

**6.** Produit pharmaceutique selon la revendication 1, comprenant du 4'-cyano-α',α',α'-trifluoro-3-(4-fluorophénylsulfonyl)-2-hydroxy-2-méthylpropiono-m-toluidide, ou un sel ou un solvate pharmaceutiquement acceptable de celui-ci, dans une dispersion solide comprenant un polymère entérique avec un $pK_a$ allant de 3 à 6, le produit comprenant en outre un inhibiteur de l'aromatase.

**7.** Produit pharmaceutique selon l'une des revendications 1 ou 6, dans lequel l'inhibiteur de l'aromatase est dans une dispersion solide avec le polymère entérique.

**8.** Produit pharmaceutique selon l'une des revendications 1, 6 ou 7, dans lequel l'inhibiteur de l'aromatase est choisi parmi l'anastrazole, le létrozole et l'exémestane, ou un sel ou un solvate pharmaceutiquement acceptable de ceux-ci.

**9.** Produit pharmaceutique selon l'une des revendications 1, 6, 7 ou 8, dans lequel le 4'-cyano-α',α',α'-trifluoro-3-(4-fluorophénylsulfonyl)-2-hydroxy-2-méthylpropiono-m-toluidide et l'inhibiteur de l'aromatase sont présents dans un rapport de 25 à 350 pour 0,005 à 100, respectivement.

**10.** Produit pharmaceutique selon l'une quelconque des revendications précédentes, dans lequel plus de 50 % du 4'-cyano-α',α',α'-trifluoro-3-(4-fluorophénylsulfonyl)-2-hydroxy-2-méthylpropiono-m-toluidide est présent sous la forme de l'énantiomère R.

**11.** Produit pharmaceutique selon la revendication 9, dans lequel plus de 50 %, 60 % et plus, 70 % et plus, 80 % et plus, 85 % et plus, 90 % et plus, 95 % et plus, 98 % et plus, 99 % et plus ou essentiellement 100 % du 4'-cyano-α',α',α'-trifluoro-3-(4-fluorophénylsulfonyl)-2-hydroxy-2-méthylpropiono-m-toluidide est présent sous la forme de l'énantiomère R.

**12.** Produit pharmaceutique selon l'une quelconque des revendications précédentes, dans lequel le polymère entérique est choisi parmi le groupe constitué par : l'acétate succinate d'hydroxypropylméthylcellulose (HPMCAS), l'acétate phtalate d'hydroxypropylméthylcellulose, l'acétate d'hydroxypropylméthylcellulose, le succinate d'hydroxypropylméthylcellulose, un copolymère d'acide méthacrylique, l'acétate phtalate de polyvinyle (PVAP), l'acétate phtalate de cellulose (CAP), l'acétate phtalate de méthylcellulose, l'acétate phtalate d'éthylcellulose, l'acétate phtalate d'hydroxypropylcellulose, le phtalate d'hydroxypropylméthylcellulose (HPMCP), le proprionate phtalate de cellulose, le butyrate phtalate d'hydroxypropylcellulose, l'acétate phtalate succinate d'hydroxypropylcellulose, le trimellitate d'hydroxypropylméthylcellulose, l'acétate trimellitate de cellulose (CAT), l'acétate trimellitate de méthylcellulose, l'acétate trimellitate d'éthylcellulose, l'acétate trimellitate d'hydroxypropylcellulose, l'acétate trimellitate d'hydroxypropylméthylcellulose, l'acétate trimellitate succinate d'hydroxypropylcellulose, le propionate trimellitate de cellulose, le butyrate trimellitate de cellulose, l'acétate téréphtalate de cellulose et l'acétate isophtalate de cellulose, ou toute combinaison de ceux-ci.

**13.** Produit pharmaceutique selon la revendication 12, dans lequel le polymère entérique est choisi parmi le groupe constitué par : du HPMCP de type HP-50, du HPMCP de type HP-55, du HPMCP de type HP-55S, du HPMCAS

de type AS-LF, du HPMCAS de type AS-MF, du HPMCAS de type AS-HF, du HPMCAS de type AS-LG, du HPMCAS de type AS-MG, du HPMCAS de type AS-HG, un copolymère d'acide méthacrylique de type A et un copolymère d'acide méthacrylique de type B.

**14.** Produit pharmaceutique selon la revendication 13, dans lequel le polymère entérique est choisi parmi le groupe constitué par : du HPMCP de type HP-55S, du HPMCAS de type AS-LG et un copolymère d'acide méthacrylique de type A.

**15.** Produit pharmaceutique selon l'une quelconque des revendications précédentes, dans lequel le rapport en poids du 4'-cyano-α',α',α'-trifluoro-3-(4-fluorophénylsulfonyl)-2-hydroxy-2-méthylpropiono-m-toluidide au polymère entérique varie de 1:0,25 à 1:10.

**16.** Produit pharmaceutique selon l'une quelconque des revendications précédentes, dans lequel la dispersion solide comprend un agent mouillant.

**17.** Produit pharmaceutique selon l'une des revendications 1 ou 2, sous forme d'une dose comprenant de 25 à 1000 mg de 4'-cyano-α',α',α'-trifluoro-3-(4-fluorophénylsulfonyl)-2-hydroxy-2-méthylpropiono-m-toluidide, la dose comprenant en outre un antioestrogène.

**18.** Produit selon la revendication 17, dans lequel l'antioestrogène est tel que défini dans l'une des revendications 3 ou 4.

**19.** Produit selon l'une des revendications 17 ou 18, dans lequel l'antioestrogène est présent en une quantité de 0,5 à 200 mg.

**20.** Produit pharmaceutique selon l'une des revendications 1 ou 6, sous forme d'une dose comprenant de 25 à 1000 mg de 4'-cyano-α',α',α'-trifluoro-3-(4-fluorophénylsulfonyl)-2-hydroxy-2-méthylpropiono-m-toluidide, la dose comprenant en outre un inhibiteur de l'aromatase.

**21.** Produit selon la revendication 20, dans lequel l'inhibiteur de l'aromatase est tel que défini dans l'une des revendications 7 ou 8.

**22.** Produit selon l'une des revendications 20 ou 21, dans lequel l'inhibiteur de l'aromatase est présent en une quantité de 0,005 à 200 mg.

**23.** Produit selon l'une quelconque des revendications 17 à 22, dans lequel plus de 50 % du 4'-cyano-α',α',α'-trifluoro-3-(4-fluorophénylsulfonyl)-2-hydroxy-2-méthylpropiono-m-toluidide est présent sous la forme de l'énantiomère R.

**24.** Produit selon la revendication 23, dans lequel plus de 50 %, 60 % et plus, 70 % et plus, 80 % et plus, 85 % et plus, 90 % et plus, 95 % et plus, 98 % et plus, 99 % et plus ou essentiellement 100 % du 4'-cyano-α',α',α'-trifluoro-3-(4-fluorophénylsulfonyl)-2-hydroxy-2-méthylpropiono-m-toluidide est présent sous la forme de l'énantiomère R.

**25.** Produit selon l'une quelconque des revendications 17 à 24, dans lequel le polymère entérique est tel que défini dans l'une quelconque des revendications 11 à 13.

**26.** Produit selon l'une quelconque des revendications 17 à 25, dans lequel le rapport en poids du 4'-cyano-α',α',α'-trifluoro-3-(4-fluorophénylsulfonyl)-2-hydroxy-2-méthylpropiono-m-toluidide au polymère entérique varie de 1:0,25 à 1:10.

**27.** Produit selon l'une quelconque des revendications 17 à 26, dans lequel la dispersion solide comprend un agent mouillant.

**28.** Utilisation dans la fabrication d'un produit pharmaceutique d'un antioestrogène et du 4'-cyano-α',α',α'-trifluoro-3-(4-fluorophénylsulfonyl)-2-hydroxy-2-méthylpropiono-m-toluidide ou d'un sel ou d'un solvate pharmaceutiquement acceptable de celui-ci, pour une administration simultanée ou séquentielle à un patient, pour augmenter la biodisponibilité du 4'-cyano-α',α',α'-trifluoro-3-(4-fluorophénylsulfonyl)-2-hydroxy-2-méthylpropiono-m-toluidide chez le patient et pour le traitement et/ou la prévention d'au moins un effet secondaire choisi parmi la gynécomastie, la sensibilité des seins, les bouffées de chaleur, l'impuissance et la réduction de la libido, le 4'-cyano-α',α',α'-trifluoro-3-(4-fluorophénylsulfonyl)-2-hydroxy-2-méthylpropiono-m-toluidide étant dans une dispersion solide avec un poly-

mère entérique ayant un $pK_a$ allant de 3 à 6.

**29.** Utilisation dans la fabrication d'un produit pharmaceutique d'un antioestrogène et du 4'-cyano-α',α',α'-trifluoro-3-(4-fluorophénylsulfonyl)-2-hydroxy-2-méthylpropiono-m-toluidide ou d'un sel ou d'un solvate pharmaceutiquement acceptable de celui-ci, pour une administration simultanée ou séquentielle à des patients, pour réduire la variabilité, d'un patient à l'autre, des concentrations plasmatiques du 4'-cyano-α',α',α'-trifluoro-3-(4-fluorophénylsulfonyl)-2-hydroxy-2-méthylpropiono-m-toluidide chez le patient, et pour le traitement et/ou la prévention d'au moins un effet secondaire choisi parmi la gynécomastie, la sensibilité des seins, les bouffées de chaleur, l'impuissance et la réduction de la libido, le 4'-cyano-α',α',α'-trifluoro-3-(4-fluorophénylsulfonyl)-2-hydroxy-2-méthylpropiono-m-toluidide étant dans une dispersion solide avec un polymère entérique ayant un $pK_a$ allant de 3 à 6.

**30.** Utilisation dans la fabrication d'un produit pharmaceutique d'un antioestrogène et du 4'-cyano-α',α',α'-trifluoro-3-(4-fluorophénylsulfonyl)-2-hydroxy-2-méthylpropiono-m-toluidide ou d'un sel ou d'un solvate pharmaceutiquement acceptable de celui-ci, pour une administration simultanée ou séquentielle à un patient, pour le traitement et/ou la réduction du risque d'un cancer de la prostate chez le patient et pour le traitement et/ou la prévention d'au moins un effet secondaire choisi parmi la gynécomastie, la sensibilité des seins, les bouffées de chaleur, l'impuissance et la réduction de la libido, le 4'-cyano-α',α',α'-trifluoro-3-(4-fluorophénylsulfonyl)-2-hydroxy-2-méthylpropiono-m-toluidide étant dans une dispersion solide avec un polymère entérique ayant un $pK_a$ allant de 3 à 6.

**31.** Utilisation selon l'une des revendications 28, 29 ou 30, dans lequel l'antioestrogène est du tamoxifène ou un sel ou un solvate pharmaceutiquement acceptable de celui-ci.

**32.** Utilisation selon l'une des revendications 28, 29, 30 ou 31, dans laquelle le 4'-cyano-α',α',α'-trifluoro-3-(4-fluorophénylsulfonyl)-2-hydroxy-2-méthylpropiono-m-toluidide et l'antioestrogène sont présents dans un rapport de 25 à 350 pour 0,5 à 100, respectivement.

**33.** Utilisation dans la fabrication d'un produit pharmaceutique d'un inhibiteur de l'aromatase et du 4'-cyano-α',α',α'-trifluoro-3-(4-fluorophénylsulfonyl)-2-hydroxy-2-méthylpropiono-m-toluidide ou d'un sel ou d'un solvate pharmaceutiquement acceptable de celui-ci, pour une administration simultanée ou séquentielle à un patient, pour augmenter la biodisponibilité du 4'-cyano-α',α',α'-trifluoro-3-(4-fluorophénylsulfonyl)-2-hydroxy-2-méthylpropiono-m-toluidide chez le patient et pour le traitement et/ou la prévention d'au moins un effet secondaire choisi parmi la gynécomastie, la sensibilité des seins, les bouffées de chaleur, l'impuissance et la réduction de la libido, le 4'-cyano-α',α',α'-trifluoro-3-(4-fluorophénylsulfonyl)-2-hydroxy-2-méthylpropiono-m-toluidide étant dans une dispersion solide avec un polymère entérique ayant un $pK_a$ allant de 3 à 6.

**34.** Utilisation dans la fabrication d'un produit pharmaceutique d'un inhibiteur de l'aromatase et du 4'-cyano-α',α',α'-trifluoro-3-(4-fluorophénylsulfonyl)-2-hydroxy-2-méthylpropiono-m-toluidide ou d'un sel ou d'un solvate pharmaceutiquement acceptable de celui-ci, pour une administration simultanée ou séquentielle à des patients, pour réduire la variabilité, d'un patient à l'autre, des concentrations plasmatiques du 4'-cyano-α',α',α'-trifluoro-3-(4-fluorophénylsulfonyl)-2-hydroxy-2-méthylpropiono-m-toluidide chez le patient, et pour le traitement et/ou la prévention d'au moins un effet secondaire choisi parmi la gynécomastie, la sensibilité des seins, les bouffées de chaleur, l'impuissance et la réduction de la libido, le 4'-cyano-α',α',α'-trifluoro-3-(4-fluorophénylsulfonyl)-2-hydroxy-2-méthylpropiono-m-toluidide étant dans une dispersion solide avec un polymère entérique ayant un $pK_a$ allant de 3 à 6.

**35.** Utilisation dans la fabrication d'un produit pharmaceutique d'un inhibiteur de l'aromatase et du 4'-cyano-α',α',α'-trifluoro-3-(4-fluorophénylsulfonyl)-2-hydroxy-2-méthylpropiono-m-toluidide ou d'un sel ou d'un solvate pharmaceutiquement acceptable de celui-ci, pour une administration simultanée ou séquentielle à un patient, pour le traitement et/ou la réduction du risque d'un cancer de la prostate chez le patient et pour le traitement et/ou la prévention d'au moins un effet secondaire choisi parmi la gynécomastie, la sensibilité des seins, les bouffées de chaleur, l'impuissance et la réduction de la libido, le 4'-cyano-α',α',α'-trifluoro-3-(4-fluorophénylsulfonyl)-2-hydroxy-2-méthylpropiono-m-toluidide étant dans une dispersion solide avec un polymère entérique ayant un $pK_a$ allant de 3 à 6.

**36.** Utilisation selon l'une quelconque des revendications 33 à 35, dans laquelle l'inhibiteur de l'aromatase est choisi parmi l'anastrazole, le létrozole et l'exémestane, ou un sel ou un solvate pharmaceutiquement acceptable de ceux-ci.

**37.** Utilisation selon l'une quelconque des revendications 33 à 36, dans laquelle le 4'-cyano-α',α',α'-trifluoro-3-(4-fluorophénylsulfonyl)-2-hydroxy-2-méthylpropiono-m-toluidide et l'inhibiteur de l'aromatase sont présents dans un rapport de 25 à 350 pour 0,005 à 100, respectivement.

**38.** Utilisation selon l'une quelconque des revendications 28 à 37, dans laquelle plus de 50 %, 60 % et plus, 70 % et plus, 80 % et plus, 85 % et plus, 90 % et plus, 95 % et plus, 98 % et plus, 99 % et plus ou essentiellement 100 % du 4'-cyano-α',α',α'-trifluoro-3-(4-fluorophénylsulfonyl)-2-hydroxy-2-méthylpropiono-m-toluidide est présent sous la forme de l'énantiomère R.

**39.** Produit pharmaceutique selon la revendication 1, comprenant du 4'-cyano-α',α',α'-trifluoro-3-(4-fluorophénylsulfonyl)-2-hydroxy-2-méthylpropiono-m-toluidide, ou un sel ou un solvate pharmaceutiquement acceptable de celui-ci, dans une dispersion solide comprenant du HPMCP de type HP-55S, le produit comprenant en outre du tamoxifène.

**40.** Produit pharmaceutique selon la revendication 39, dans lequel plus de 50 %, 60 % et plus, 70 % et plus, 80 % et plus, 85 % et plus, 90 % et plus, 95 % et plus, 98 % et plus, 99 % et plus ou essentiellement 100 % du 4'-cyano-α',α',α'-trifluoro-3-(4-fluorophénylsulfonyl)-2-hydroxy-2-méthylpropiono-m-toluidide est présent sous la forme de l'énantiomère R.

**41.** Produit selon l'une quelconque des revendications 1 à 27, 39 et 40, dans lequel au moins 30 %, 40 %, 50 %, 75 %, 90 %, 95 % ou 99 % du 4'-cyano-α',α',α'-trifluoro-3-(4-fluorophénylsulfonyl)-2-hydroxy-2-méthylpropiono-m-toluidide est présent sous une forme amorphe.

**42.** Utilisation selon l'une quelconque des revendications 28 à 38, dans laquelle au moins 30 %, 40 %, 50 %, 75 %, 90 %, 95 % ou 99 % du 4'-cyano-α',α',α'-trifluoro-3-(4-fluorophénylsulfonyl)-2-hydroxy-2-méthylpropiono-m-toluidide est présent sous une forme amorphe.

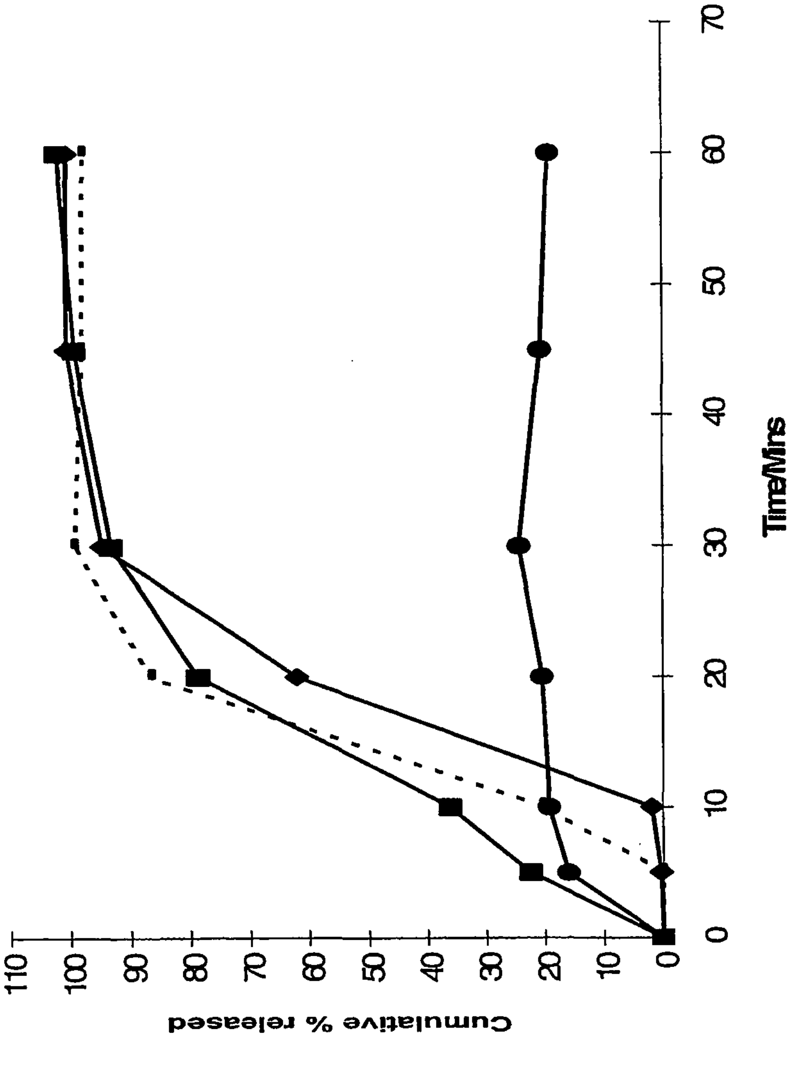
Cumulative % released
0
10
20
30
40
50
60
70
80
90
100
110
Time/Mins
0
10
20
30
40
50
60
70

Figure 1

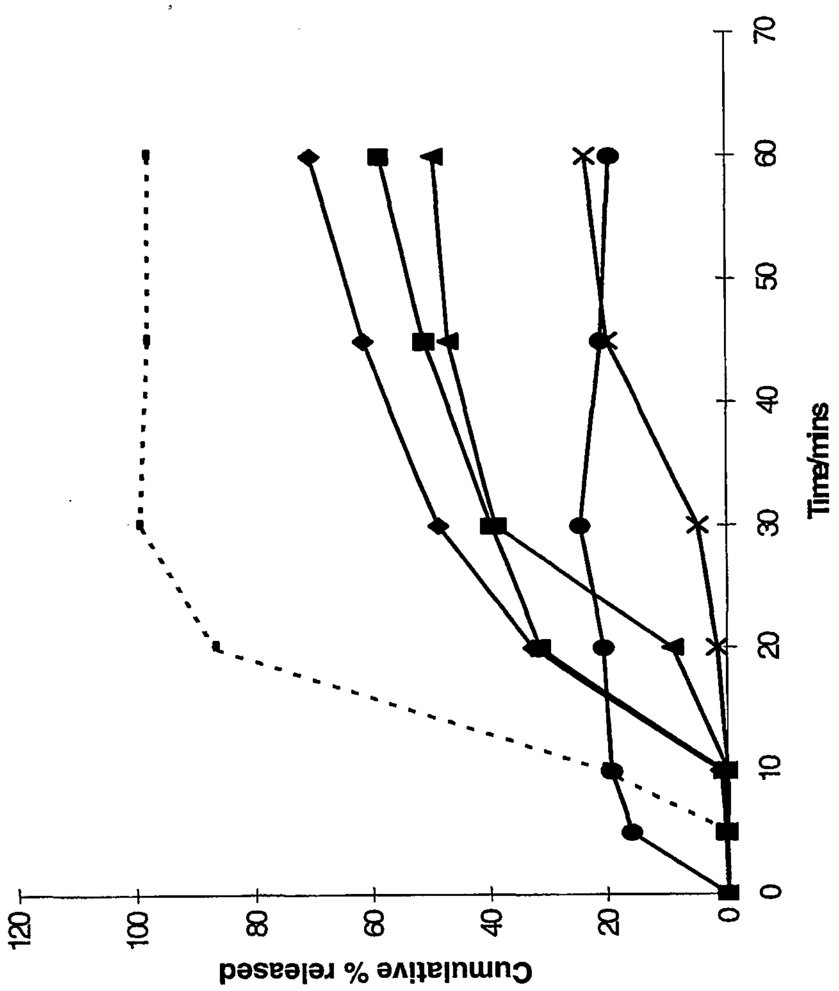
Cumulative % released
120
100
80
60
40
20
0
0
10
20
30
40
50
60
70
Time/mins

Figure 2

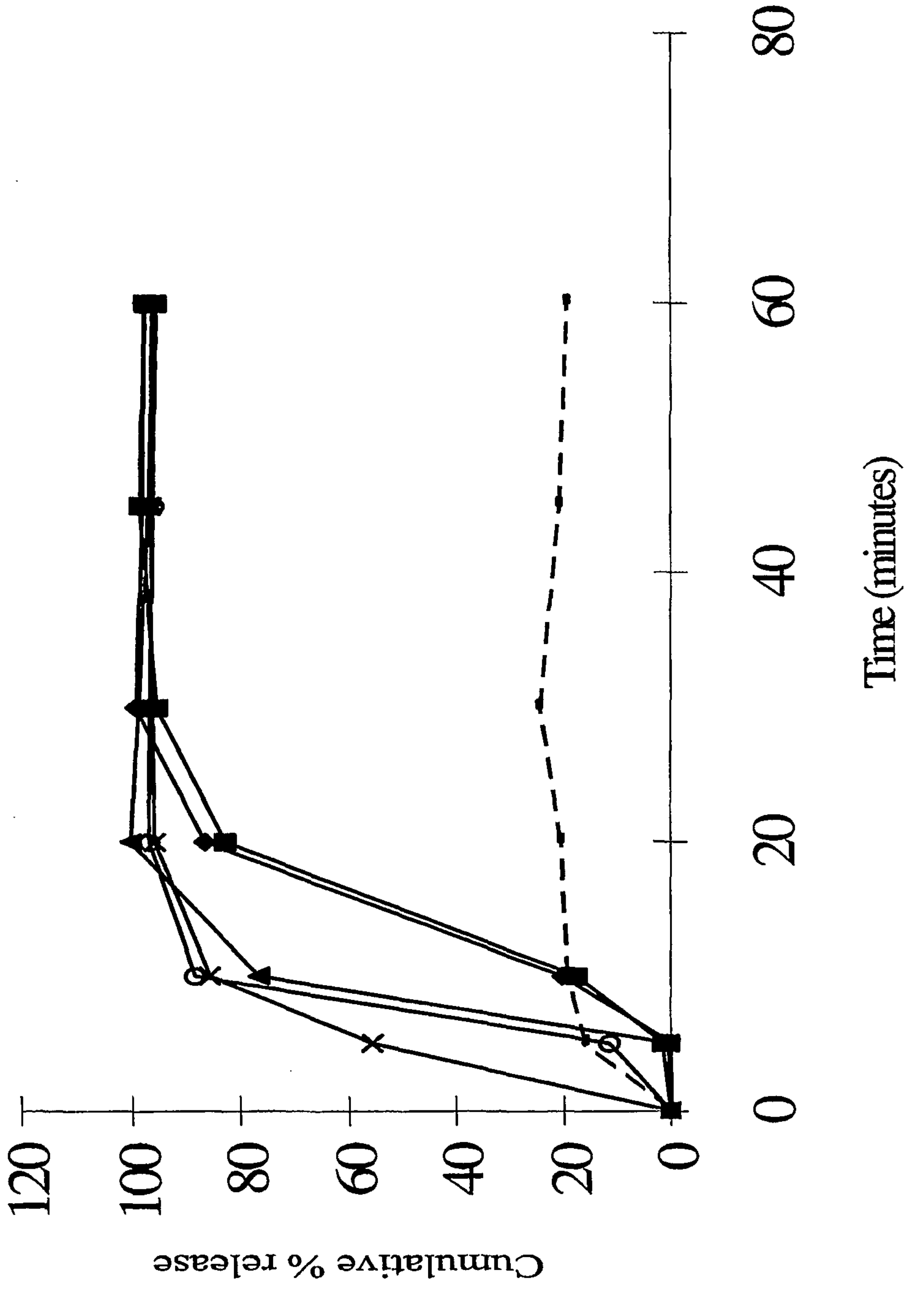

Cumulative % release
120
100
80
60
40
20
0
0
20
40
60
80
Time (minutes)


**Figure 3**

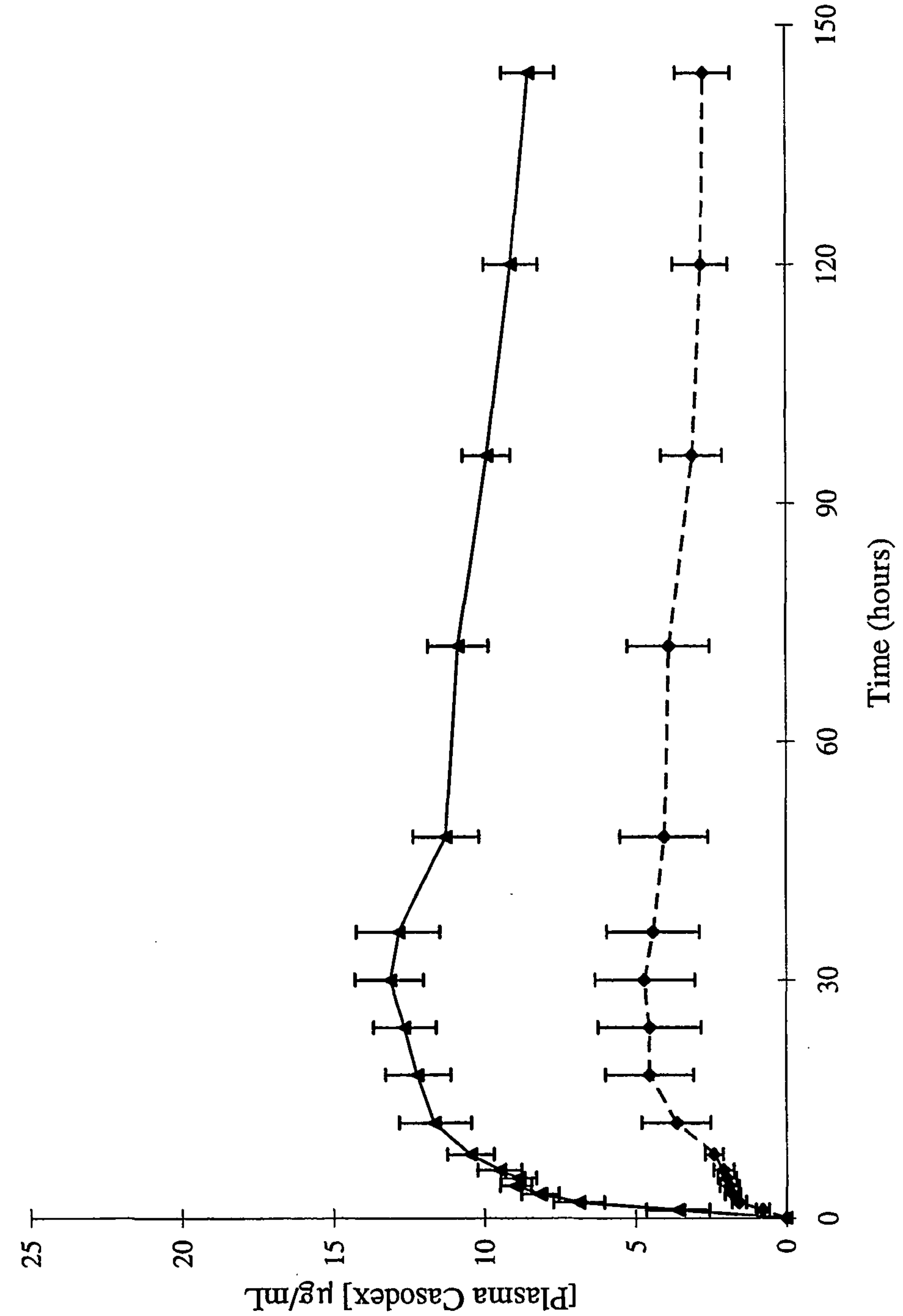


**Figure 4**

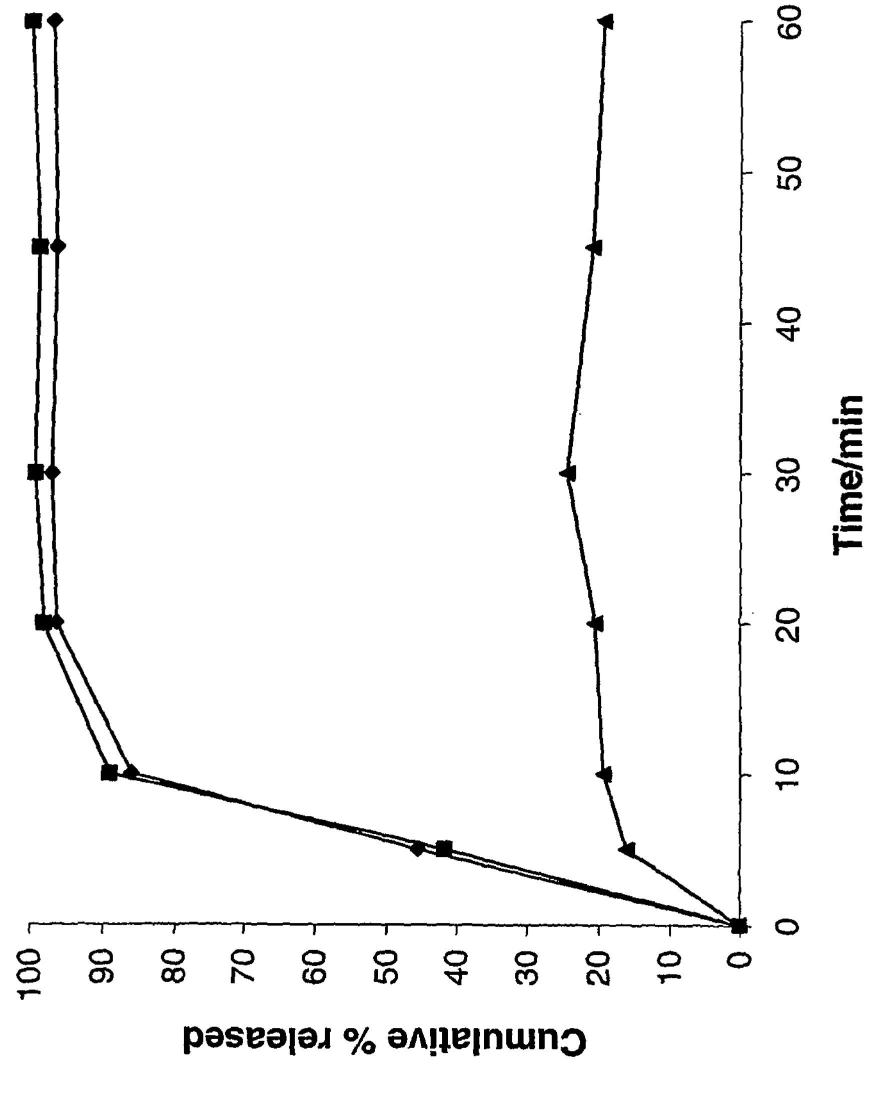
Cumulative % released
0
10
20
30
40
50
60
70
80
90
100
Time/min
0
10
20
30
40
50
60

Figure 5

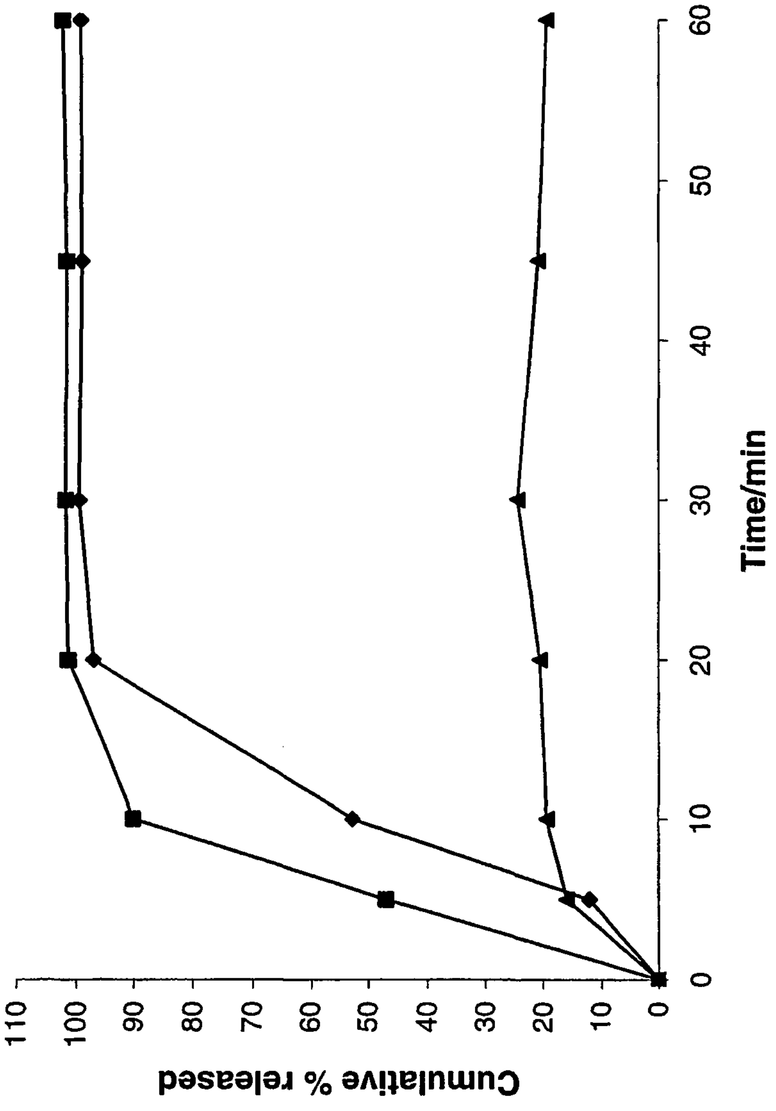
Cumulative % released
0
10
20
30
40
50
60
70
80
90
100
110
Time/min
0
10
20
30
40
50
60

Figure 6

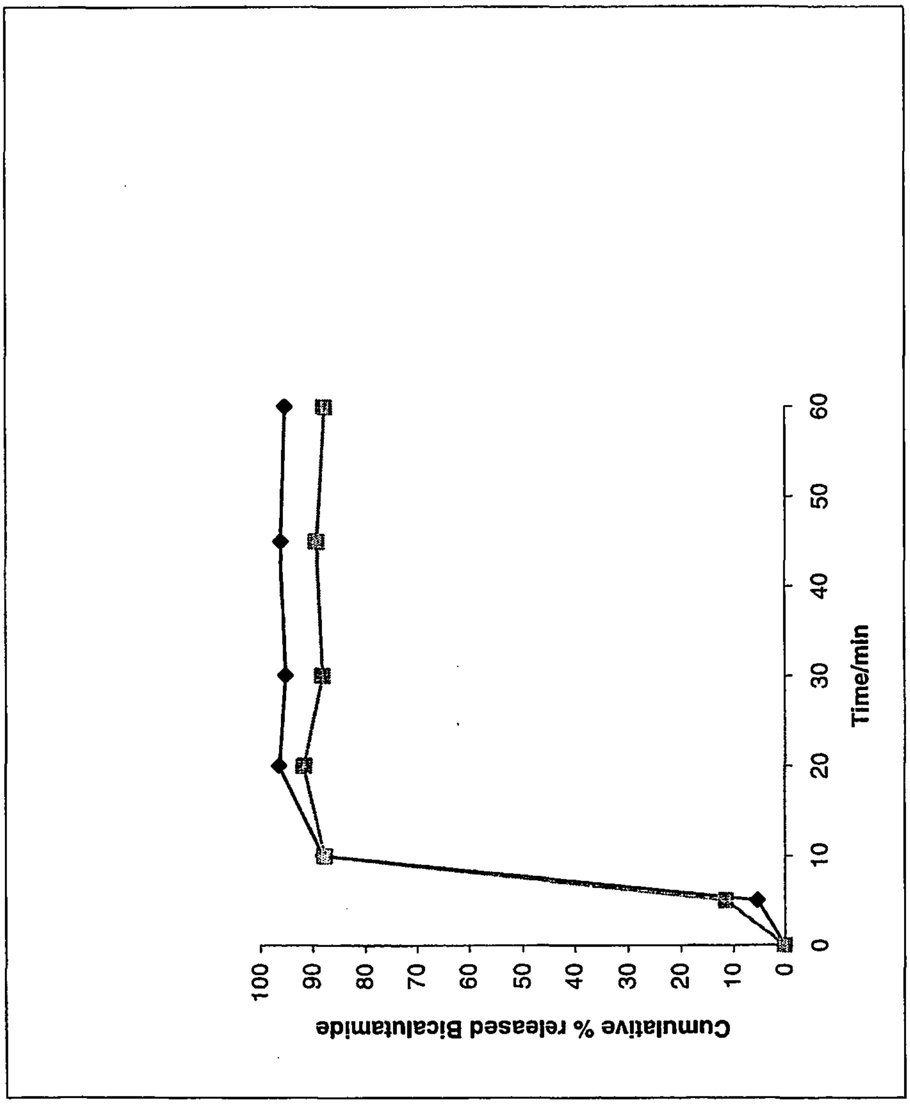
Cumulative % released Bicalutamide
100
90
80
70
60
50
40
30
20
10
0
0
10
20
30
40
50
60
Time/min

**Figure 7**

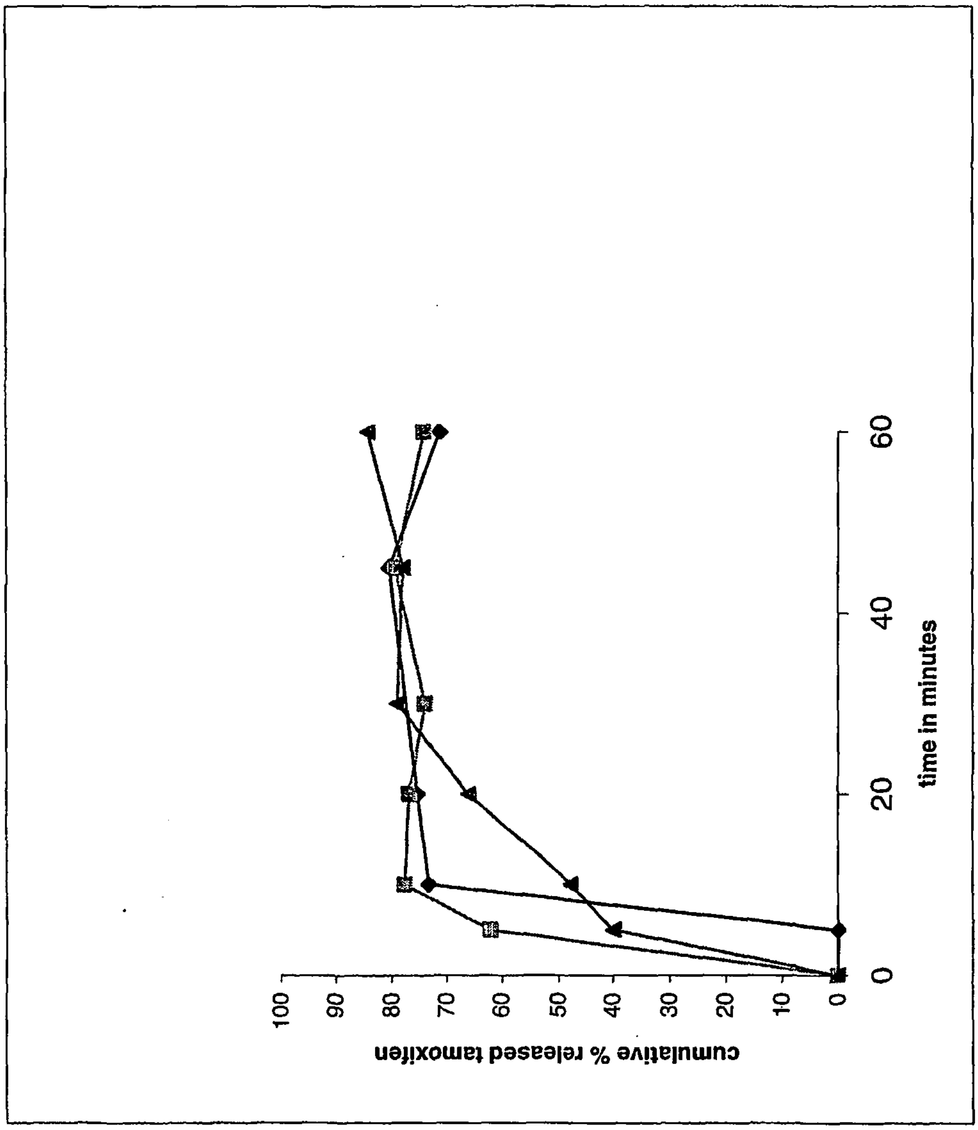
cumulative % released tamoxifen
100
90
80
70
60
50
40
30
20
10
0
0
20
40
60
time in minutes

Figure 8

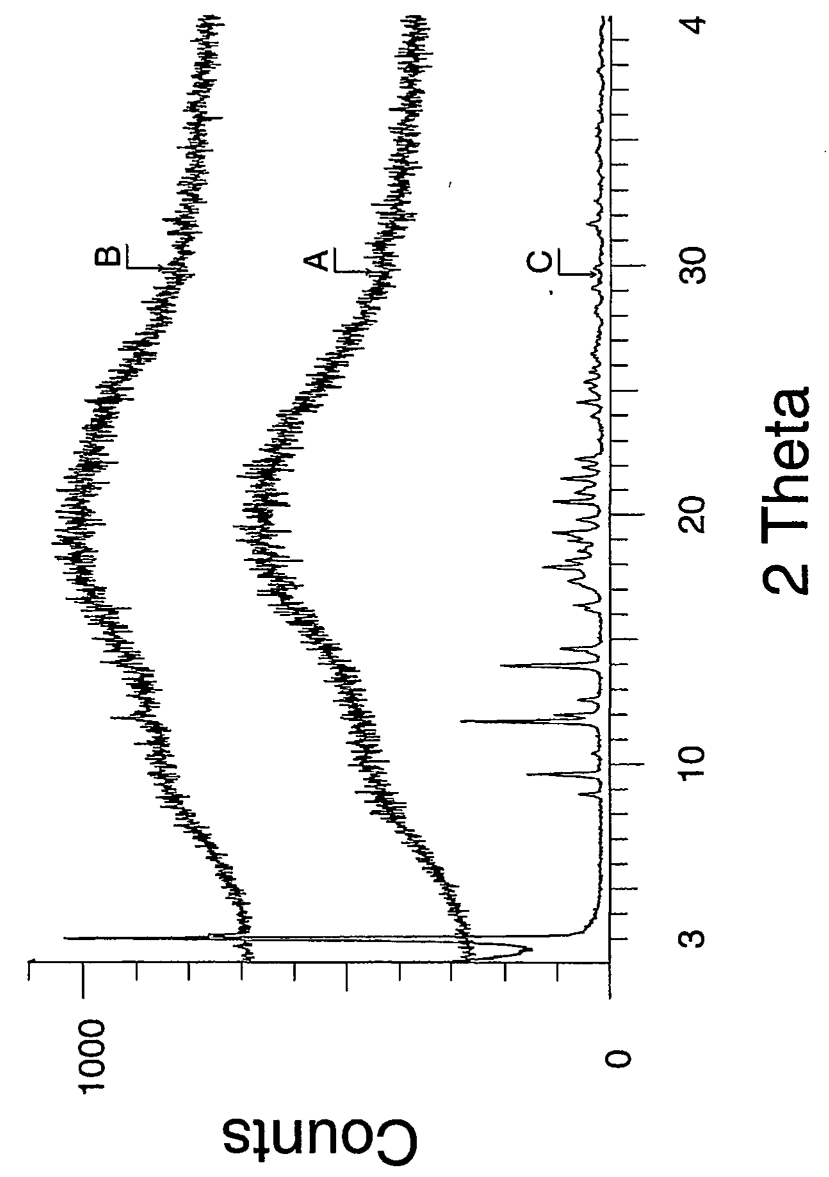


Figure 9